# EUROPEAN PATENT SPECIFICATION

(11) **EP 3 893 826 B1**
(45) Date of publication and mention of the grant of the patent: **22.11.2023**
(21) Application number: 19895588.2
(22) Date of filing: 10.12.2019
(51) Int. Cl.: A61F 13/15, B32B 5/26, B32B 7/04, A61F 13/511, A61F 13/514, B32B 5/02, B32B 5/04, B32B 5/06, B32B 7/09, B32B 7/12

(54) **A MULTI-LAYER TEXTILE ASSEMBLY**
MEHRLAGIGES TEXTILMATERIAL
ENSEMBLE TEXTILE MULTICOUCHE

(30) Priority: 10.12.2018 NZ 18749123; 30.05.2019 AU 2019901862
(43) Date of publication of application: 20.10.2021
(73) Proprietor: RSD Holdings Limited, Auckland, 1023 (NZ)
(72) Inventor: RIHA-SCOTT, Frantisek, Auckland, 0626 (NZ)
(74) Representative: Algemeen Octrooi- en Merkenbureau B.V.
(86) International application number: PCT/IB2019/060587
(87) International publication number: WO 2020/121176

(56) References cited:
- WO-A1-2012/087292
- WO-A1-2014/184640
- WO-A2-2004/079064
- US-A- 5 290 269
- US-A1- 2006 100 597
- US-A1- 2012 276 332
- US-B2- 7 214 120

## Description

### FIELD OF THE INVENTION

The present invention relates to a multi-layer textile assembly. In particular though not solely, the present invention relates to a multi-layer textile assembly and its use in garments such as but not limited to absorbent garment and undergarments such as but not limited to being used for people having incontinence (bladder leakage), menstruation or any other bodily discharge.

### BACKGROUND TO THE INVENTION

Currently there are mostly disposable absorbent articles in the market available to absorb bodily liquids and protect wearers from undesired embarrassment caused by bladder leakage, menstruation or other bodily discharges. Consumers in today's world are seeking higher performance products that are also eco-friendly and chemical free. At least for such reasons, there is a strong need for performance textiles that are absorbent, washable, waterproof/leakproof, moisture retaining. Most current conventional textile materials or natural fibres such as cotton may have some degree of moisture absorbent properties but a person wearing an article of clothing made from such material and that has absorbed some moisture, may find themselves to be in a cool and moist state. Furthermore, conventional textile materials such as cotton do not achieve desired moisture retention when under pressure and may have a very low liquid or moisture absorbency. Also, use of natural fibres in conventional textiles means such textile materials are not odour protective and might not have anti-bacterial properties and may therefore support bacteria growth causing undesired odour, skin irritations and other health problems.

The foregoing drawbacks are just some of the problems encountered with conventional textiles or textile assemblies.

US-A1-5,290,269 describes a knit terry fabric having hydrophobic/hydrophilic characteristics providing hygienic panels for washable, reusable, incontinent pads and diapers comprising a non-woven fabric, stiffener sheet employed in an incontinent pad to resist its being folded in use as well as a Y-shaped configuration.

US2006/100597A1 describes a multi-layer moisture management fabric composite including a three-dimensional fabric spacer, which forms a first layer of the fabric composite with a first facing adapted for residing nearest the skin, a second facing, and an intermediate spacer yarn interconnecting the first and second facings.

WO2012/087292A1 describes a liner for use in skin-contacting garments and other objects that contact the skin, the liner having a moisture-wicking layer formed from a non-shearing, moisture-vapor permeable woven fabric of synthetic filament yarns which is treated with an anti-microbial substance.

### OBJECT OF THE INVENTION

It is an object of the present invention to provide a multi-layer textile assembly that overcomes or at least partially ameliorates some of the abovementioned disadvantages or which at least provides the public with a useful choice.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### SUMMARY OF THE INVENTION

In a first aspect, the invention broadly resides in a multi-layer liquid absorbing and retaining textile assembly as outlined in independent claim 1 with additional details outlined in dependent claims 2-5 (herein after also referred to as "textile fabric"), the textile assembly comprising:
a first layer made of or constructed as a pique knitted, one-way wicking material,
a second layer made of or constructed as a microfibre material, and
an intermediate layer made of or constructed as a warp knitted spacer fabric having a plurality of sections with at least one of the plurality of sections being proximal to the first layer and at least another one of the plurality of sections being proximal to the second layer,
the intermediate layer being positioned between the first layer and the second layer and is adapted to interconnect the first layer and the second layer,
the first layer being adapted to transfer liquid from a first side of the textile assembly to the intermediate layer, the intermediate layer is adapted to receive the liquid from the first layer and the second layer is adapted to retain the liquid at or proximal to a second side of the textile assembly that is oppositely facing to the first side.

In a further aspect, the invention pertains to a method of making a multi-layer liquid absorbing and retaining textile assembly for absorbing and retaining liquid emitted from a human body, as defined in claim 6.

At least one and preferably each of the plurality of sections has a knitting density that is different from a knitting density of another section or sections.

In one embodiment the The sections are of a multi-layer assembly The plurality of sections comprise at least three sections that are a first section, a second section and a middle section with the middle section interconnecting the first and second sections, the first section being proximal to the first layer and the second section being proximal to the second layer, wherein the knitting density of the first, second and middle sections are high to low in the following order:
i. the second section
ii. the first section
iii. the middle section.

The liquid transferred from the first layer is adapted to be received by the first section, middle section and the second section of the intermediate layer in the following sequential order before being transferred to the second layer:
i. the first section
ii. the middle section
iii. the second section.

The first section is warp knitted to form a plurality of pores to receive the liquid from the first layer.

The second section is knitted in a one-way knitting pattern to help prevent liquid in the second layer from flowing back to the intermediate layer.

The middle section is warp knitted to form substantially vertically oriented fibres.

The second section is warp knitted to form a plurality of pores to receive the liquid from the middle section, wherein pores of the second section are smaller in size than the pores of the first section.

In one embodiment, when the textile assembly is twisted, wrung or squeezed, the substantially vertically oriented fibres (or a tension in the substantially vertically oriented fibres) causes at least a portion of the second section to be pulled thereby causing at least some of the pores of the second section to be wider and/or knitting density of the second section to be distorted for at least some of the liquid to escape from the textile assembly.

In one embodiment, the first layer comprises or is made from at least one hydrophobic fibre material.

In one embodiment, the microfibre material comprises a plurality of gaps in between for collecting liquid.

In one embodiment, the intermediate layer is directly or indirectly affixed to the first layer and the second layer thereby interconnecting the first layer and the second layer.

In one embodiment, the textile assembly is antibacterial and/or hypoallergenic.

In one embodiment, the textile assembly is between 4mm to 6mm in thickness.

In one embodiment, the textile assembly is stretchable (preferably two or four way stretchable).

In one embodiment, the first layer and the second layer are laminated, stitched, attached or otherwise adhered to at least the intermediate layer.

In one embodiment, the first layer, the second layer and the intermediate layer are assembled to each other using adhesive.

In one embodiment, the first, second and intermediate layers are co-extensive to each other.

In one embodiment, the adhesive forms an adhesive layer having spacing or gap(s) to allow the textile assembly to have wicking properties.

In one embodiment, the weight of the textile assembly is or about 410 grams per square meter (gsm).

In one embodiment at least one (and preferably all) of the first layer, the second layer and the intermediate layer have hypoallergenic properties.

The first layer is formed as a pique knitted mesh.

In one embodiment, the first layer is antibacterial.

In one embodiment, the first layer is odour resistant.

In one embodiment, the first layer has a (fast) wicking and drying properties.

In one embodiment, the first layer comprises a polyester.

In one embodiment, the first layer comprises polyamide, polyester or a combination thereof.

In one embodiment, the first layer comprises only a polyester.

In one embodiment, the first layer is made from a material comprising a polyester.

In one embodiment, the first layer comprises 37.2% or about 37.2% polyester or is made from a material comprising 37.2% or about 37.2 % polyester.

In one embodiment, the first layer is made from a material that comprises spandex/elastane.

In one embodiment, the first layer comprises spandex/elastane.

In one embodiment, the first layer comprises 6% spandex/elastane or is made from a material comprising 6% spandex/elastane.

In one embodiment, the weight of the first layer is 170 grams per square meter (gsm).

In one embodiment, the microfibre material is a stretch microfibre material.

In one embodiment, the second layer comprises a highly absorbent and liquid retaining microfibre towelling or is made from a highly absorbent and liquid retaining microfibre towelling.

In one embodiment, the second layer comprises a polyester.

In one embodiment, the second layer comprises only a polyester.

In one embodiment, the second layer is made from a material comprising a polyester.

In one embodiment, the second layer is made from a material comprising 37.2% polyester or about 37.2% polyester.

In one embodiment, the second layer comprises 37.2% polyester or about 37.2% polyester.

In one embodiment, the second layer is made from a material comprising spandex/elastane.

In one embodiment, the second layer material comprises spandex/elastane.

In one embodiment, the second layer is made from a material comprising 6% spandex/elastane.

In one embodiment, the second layer comprises 6% spandex/elastane.

In one embodiment, the second layer comprises a polyamide.

In one embodiment, the second layer is made from a material comprising a polyamide.

In one embodiment, the second layer is made from a material comprising 12% or about 12% spandex/elastane.

In one embodiment, the second layer comprises 12% or about 12% spandex/elastane.

In one embodiment, the second layer is made from a material comprising 12% or about 12% polyamide.

In one embodiment, the second layer comprises 12% or about 12% polyamide.

In one embodiment, the second layer is made from a material comprising 76% or about 76% polyester.

In one embodiment, the second layer comprises 76% or about 76% polyester.

In one embodiment the second layer comprises 76% Polyester, 12% polyamine and 12% elastane.

In one embodiment, the microfibre material comprises a liquid resistant oil print to retain liquids.

In one embodiment, the microfibre material is 2mm or about 2mm in thickness.

In one embodiment, the intermediate layer is made from a nylon.

In one embodiment, the intermediate layer comprises nylon.

In one embodiment, the intermediate layer is made from a material comprising a nylon.

In one embodiment, the intermediate layer is made from a material comprising 19.6% nylon or about 19.6% nylon.

In one embodiment, the intermediate layer comprises 19.6% nylon or about 19.6% nylon.

In one embodiment, the intermediate layer comprises 74.4% polyester, 19.6% polyamide (nylon) and 6% elastane.

In one embodiment, the intermediate layer is 2mm or about 2mm in thickness.

In one embodiment, the intermediate layer is 1.3 mm or about 1.3 mm in thickness.

In one embodiment, the textile assembly is adapted to retain liquid when external force or pressure is applied from a direction that is orthogonal or substantially orthogonal to the surface area the textile assembly.

In one embodiment, the textile assembly is adapted to help retain liquid unless the textile assembly is twisted, wrung or squeezed to an extent that the layers are compacted. In other words, the textile assembly is adapted to release liquid or only release liquid when the textile assembly is twisted, wrung or squeezed.

In one embodiment, the textile assembly is adapted to retain more liquid when the textile assembly is unstressed (e.g. when lying on a flat surface without any pressure other than gravity acting on it)and adapted to retain less liquid when stress (e.g. a twisting or wringing or compacting) is applied to said assembly (such as experienced during twisting or squeezing of the textile assembly).

In one embodiment, the first second and intermediate layers are contiguous to each other.

In one embodiment, at least one layer of a material may interpose between at least one of the first and intermediate layer and the second and intermediate layer.

In one embodiment, the textile assembly comprises a leakproof layer sealing and/or trapping liquid at the second layer at least on a side of the second layer that is opposite to side of the second layer that is proximal to the intermediate layer.

In one embodiment, the leakproof layer is laminated, stitched, attached or otherwise adhered to at least the second layer.

In one embodiment, the leakproof layer is resin coated woven leakproof textile.

In one embodiment, the leakproof layer comprises or is made from 100% polyamide.

In one embodiment, the textile assembly further comprises a fourth layer located between the first layer and the intermediate layer.

In one embodiment, the first layer and the intermediate layer are laminated, stitched, attached or otherwise adhered to at least the fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the fourth layer is formed as a carbon crystal mesh.

In one embodiment, the textile assembly is able to or is incorporated with or adapted for use as part of or is a garment.

Also disclosed is a multi-layer liquid absorbing and retaining textile assembly (herein also referred to as "textile fabric") to be formed or be incorporated into a garment for absorbing and retaining liquid emitted from a human body, the textile assembly comprising:
a first layer made of or is constructed as a one-way wicking fibre material,
a second layer that is made of or is constructed of a microfibre material with a plurality of gaps for collecting and retaining liquid, and
an intermediate layer made of or constructed as a spacer fabric to receive and collect the liquid from the first layer and to transfer the collected liquid to the second layer,
the spacer fabric being warp knitted to have a plurality of sections with at least one of the plurality of sections being proximal to the first layer and at least another one of the plurality of sections being proximal to the second layer, each one of the plurality of the sections being of different knitting density from another one of the plurality of sections the intermediate layer being positioned between the first layer and the second layer interconnecting the first layer and the second layer.

In one embodiment, the plurality of sections comprises at least three sections that are a first section, a middle section and a second section with the middle section interconnecting the first and second sections and the intermediate layer is positioned between the first layer and the second layer with the first section being proximal to the first layer and the second section being proximal to the second layer.

In one embodiment the sections are of a multi-layer assembly.

In one embodiment, the first layer comprises or is made from at least a hydrophobic fibre material to draw away liquid from the human body and transfer the liquid to the intermediate layer.

In one embodiment, the second layer comprises or is made from at least a microfibre material to absorb and collect liquid from the intermediate layer.

In one embodiment, the first section is warp knitted to form a plurality of pores to receive the liquid from the first layer.

In one embodiment, the middle section is warp knitted to form substantially vertically oriented fibres.

In one embodiment, the second section is knitted in a one-way knitting pattern to help prevent liquid in the second layer from flowing back to the intermediate layer.

In one embodiment, the second section is warp knitted to form a plurality of pores to receive the liquid from the middle section, wherein size of the pores of the second section being smaller than the size of the pores of the first section.

In one embodiment, the first, second and middle sections have a knitting density from high to low in the following order:
i. the second section
ii. the first section
iii. the middle section.

In one embodiment, when the textile assembly is twisted, wrung or squeezed, the substantially vertically oriented fibres (or tension in the substantially vertically oriented fibres) causes at least a portion of the second section to be pulled thereby causing at least some of the pores of the second section to be wider and/or knitting density of the second section to be distorted for at least some of the liquid to escape from the textile assembly.

In one embodiment, the intermediate layer is directly or indirectly affixed to the first layer and the second layer thereby interconnecting the first layer and the second layer.

In one embodiment, the textile assembly is stretchable (preferably two or four way stretchable).

In one embodiment, the first layer and the second layer are laminated, stitched, attached or otherwise adhered to at least the intermediate layer.

In one embodiment, the first, second and intermediate layers are co-extensive to each other.

In one embodiment, the first layer, the second layer and the intermediate layer are affixed to each other using adhesive.

In one embodiment, the adhesive forms an adhesive layer having spacing or gap(s) to allow the textile assembly to have wicking properties.

In one embodiment, the weight of the textile assembly is or is about 410 grams per square meter (gsm).

In one embodiment, at least one (and preferably all) of the first layer, the second layer and the intermediate layer have hypoallergenic properties.

In one embodiment, the first layer is pique knitted.

In one embodiment, the first layer is formed as a pique knitted mesh.

In one embodiment, the first layer is antibacterial.

In one embodiment, the first layer is odour resistant.

In one embodiment, the first layer has a (fast) wicking and drying properties.

In one embodiment, the first layer comprises a polyester.

In one embodiment, the first layer comprises only a polyester.

In one embodiment, the first layer comprises polyamide, polyester or a combination thereof.

In one embodiment, the first layer is made from a material comprising a polyester.

In one embodiment, the first layer comprises 37.2% or about 37.2 % polyester or is made from a material comprising 37.2% or about 37.2 % polyester.

In one embodiment, the first layer is made from a material that comprises spandex/elastane.

In one embodiment, the first layer is made from a material comprising spandex/elastane.

In one embodiment, the first layer comprises 6% spandex/elastane or is made from a material comprising 6% spandex/elastane.

In one embodiment, the weight of the first layer is 170 grams per square meter (gsm).

In one embodiment, the second layer is made from a material that is a two way stretch microfibre.

In one embodiment, the second layer comprises a stretch microfibre.

In one embodiment, the second layer comprises a highly absorbent and liquid retaining microfibre towelling or is made from a highly absorbent and liquid retaining microfibre towelling.

In one embodiment, the second layer comprises a polyester.

In one embodiment, the second layer comprises only a polyester.

In one embodiment, the second layer is made from a material comprising a polyester.

In one embodiment, the second layer is made from a material comprising 37.2% polyester or about 37.2 % polyester.

In one embodiment, the second layer comprises 37.2% polyester or about 37.2% polyester.

In one embodiment, the second layer is made from a material comprising spandex/elastane.

In one embodiment, the second layer material comprises spandex/elastane.

In one embodiment, the second layer is made from a material comprising 6% spandex/elastane.

In one embodiment, the second layer comprises 6% spandex/elastane.

In one embodiment, the second layer comprises a polyamide.

In one embodiment, the second layer is made from a material comprising a polyamide.

In one embodiment, the second layer is made from a material comprising 12% or about 12% spandex/elastane.

In one embodiment, the second layer comprises 12% or about 12% spandex/elastane.

In one embodiment, the second layer is made from a material comprising 12% or about 12% polyamide.

In one embodiment, the second layer comprises 12% or about 12% polyamide.

In one embodiment, the second layer is made from a material comprising 76% or about 76% polyester.

In one embodiment, the second layer comprises 76% or about 76% polyester.

In one embodiment the second layer comprises 76% Polyester, 12% polyamine and 12% elastane.

In one embodiment, the second layer is tricot knitted.

In one embodiment, the intermediate layer is made from a nylon.

In one embodiment, the intermediate layer comprises nylon.

In one embodiment, the intermediate layer is made from a material comprising a nylon.

In one embodiment, the intermediate layer is made from a material comprising 19.6% nylon or about 19.6% nylon.

In one embodiment, the intermediate layer comprises 19.6% nylon or about 19.6% nylon.

In one embodiment, the intermediate layer comprises 74.4%Polyester, 19.6% polyamide (nylon) and 6% elastane.

In one embodiment, the intermediate layer is 2mm or about 2mm in thickness.

In one embodiment, the intermediate layer is 1.3 mm or about 1.3 mm in thickness.

In one embodiment, the textile assembly is adapted to retain liquid when external force or pressure is applied from a direction that is orthogonal or substantially orthogonal to the surface area the textile assembly.

In one embodiment, the textile assembly is adapted to retain liquid unless the textile assembly is twisted, wrung or squeezed. In other words, the textile assembly is adapted to release liquid or only release liquid when the textile assembly is twisted, wrung or squeezed.

In one embodiment, the textile assembly is adapted to retain more liquid when the textile assembly is unstressed and adapted to retain less liquid when stress is applied to said assembly (such as experienced during twisting or squeezing of the textile assembly).

In one embodiment, the first second and intermediate layers are contiguous to each other.

In one embodiment, at least one layer of a material may interpose between at least one of the first and intermediate layer and the second and intermediate layer.

In one embodiment, the textile assembly comprises a leakproof layer sealing and/or trapping liquid at the second layer at least on a side of the second layer that is opposite to side of the second layer that is proximal to the intermediate layer.

In one embodiment, the leakproof layer is laminated, stitched, attached or otherwise adhered to at least the second layer.

In one embodiment, the leakproof layer is resin coated woven leakproof textile.

In one embodiment, the leakproof layer comprises or is made from 100% polyamide.

In one embodiment, the textile assembly further comprises a fourth layer located between the first layer and the intermediate layer.

In one embodiment, the first layer and the intermediate layer are laminated, stitched, attached or otherwise adhered to at least the fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the fourth layer is formed as a carbon crystal mesh.

In one embodiment, the textile assembly is able to or is incorporated with or adapted for use as part of or is a garment.

In a third aspect, the invention resides in a multi-layer liquid absorbing and retaining textile assembly (herein also referred to as "textile fabric") for absorbing and retaining liquid emitted from a human body, the textile assembly comprising:
a first layer of a pique knitted one-way wicking configuration,
a second layer of a tricot knitted and liquid retaining configuration, and
an intermediate layer made of or constructed as a warp knitted spacer fabric and formed as plurality of sections with at least one of the plurality of sections being proximal to the first layer and at least another one of the plurality of sections being proximal to the second layer,
the intermediate layer positioned between the first layer and the second layer and is adapted to interconnect the first layer and the second layer,
wherein, the first layer is adapted to transfer liquid from a first side of the textile assembly to the intermediate layer, the intermediate layer is adapted to receive the liquid from the first layer and the second layer is adapted to retain the liquid at or proximal to a second side of the textile assembly that is oppositely facing to the first side.

In one embodiment the sections are of a multi-layer assembly.

In one embodiment, the plurality of sections comprise at least three sections that are a first section, a second section and a middle section with the middle section interconnecting the first and second sections, the first section being proximal to the first layer and the second section being proximal to the second layer, wherein the knitting density of the first, second and middle sections are high to low in the following order:
i. the second section
ii. the first section
iii. the middle section.

In one embodiment, the liquid transferred from the first layer is adapted to be received by the first section, middle section and the second section of the intermediate layer in the following sequential order before being transferred to the second layer:
i. the first section
ii. the middle section
iii. the second section.

In one embodiment, the first section is warp knitted to form a plurality of pores to receive the liquid from the first layer.

In one embodiment, the second section is knitted in a one-way knitting pattern to help prevent liquid in the second layer from flowing back to the intermediate layer.

In one embodiment, the middle section is warp knitted to form substantially vertically oriented fibres.

In one embodiment, the second section is warp knitted to form a plurality of pores to receive the liquid from the middle section, wherein pores of the second section are smaller in size than the pores of the first section.

In one embodiment, when the textile assembly is twisted, wrung or squeezed, the substantially vertically oriented fibres (or tension in the substantially vertically oriented fibres) causes at least a portion of the second section to be pulled thereby causing at least some of the pores of the second section to be wider and/or knitting density of the second section to be distorted for at least some of the liquid to escape from the textile assembly.

In one embodiment, the first layer comprises or is made from at least one hydrophobic fibre material.

In one embodiment, the second layer is made from at least a microfibre material.

In one embodiment, the microfibre material comprises a plurality of gaps in between for collecting liquid.

In one embodiment, the intermediate layer is directly or indirectly affixed to the first layer and the second layer thereby interconnecting the first layer and the second layer.

In one embodiment, the intermediate layer is directly or indirectly affixed to the first layer and the second layer thereby interconnecting the first layer and the second layer.

In one embodiment, the textile assembly is antibacterial and/or hypoallergenic.

In one embodiment, the textile assembly is between 4mm to 6mm in thickness.

In one embodiment, the textile assembly is stretchable (preferably two or four way stretchable).

In one embodiment, the first layer and the second layer are laminated, stitched, attached or otherwise adhered to at least the intermediate layer.

In some embodiment the first, second and intermediate layers are co-extensive to each other.

In one embodiment, the first layer, the second layer and the intermediate layer are affixed to each other using adhesive.

In one embodiment, the adhesive may form an adhesive layer having spacing or gap(s) to allow the textile assembly to have wicking properties.

In one embodiment, the weight of the textile assembly is or about 410 grams per square meter (gsm).

In one embodiment at least one (and preferably all) of the first layer, the second layer and the intermediate layer have hypoallergenic properties.

In one embodiment, the first layer is a pique knitted mesh.

In one embodiment, the first layer is antibacterial.

In one embodiment, the first layer is odour resistant.

In one embodiment, the first layer has a (fast) wicking and drying properties.

In one embodiment, the first layer comprises a polyester.

In one embodiment, the first layer comprises only a polyester.

In one embodiment, the first layer comprises polyamide, polyester or a combination thereof.

In one embodiment, the first layer is made from a material comprising a polyester.

In one embodiment, the first layer comprises a polyester or is made from a material comprising 37.2% or about 37.2% polyester.

In one embodiment, the first layer comprises spandex/elastane.

In one embodiment, the first layer is made from a material comprising spandex/elastane.

In one embodiment, the first layer comprises 6% spandex/elastane or is made from a material comprising 6% spandex/elastane.

In one embodiment, the weight of the first layer is 170 grams per square meter (gsm).

In one embodiment, the second layer is made from a material that is a stretch microfibre.

In one embodiment, the second layer comprises a stretch microfibre.

In one embodiment, the second layer comprises a highly absorbent and liquid retaining microfibre towelling or is made from a highly absorbent and liquid retaining microfibre towelling.

In one embodiment, the second layer comprises a polyester.

In one embodiment, the second layer comprises only a polyester

In one embodiment, the second layer is made from a material comprising a polyester.

In one embodiment, the second layer is made from a material comprising 37.2% polyester or about 37.2% polyester.

In one embodiment, the second layer comprises 37.2% polyester or about 37.2% polyester.

In one embodiment, the second layer comprises or is made from a material comprising spandex/elastane.

In one embodiment, the second layer comprises 6% spandex/elastane or is made from a material comprising 6% spandex/elastane.

In one embodiment, the second layer comprises 6% spandex/elastane.

In one embodiment, the second layer comprises a polyamide.

In one embodiment, the second layer is made from a material comprising a polyamide.

In one embodiment, the second layer is made from a material comprising 12% or about 12% spandex/elastane.

In one embodiment, the second layer comprises 12% or about 12% spandex/elastane.

In one embodiment, the second layer is made from a material comprising 12% or about 12% polyamide.

In one embodiment, the second layer comprises 12% or about 12% polyamide.

In one embodiment, the second layer is made from a material comprising 76% or about 76% polyester.

In one embodiment, the second layer comprises 76% or about 76% polyester.

In one embodiment the second layer comprises 76% polyester, 12% polyamine and 12% elastane.

In one embodiment, the second layer comprises a liquid resistant oil print to retain liquids.

In one embodiment, the second layer is 2mm or about 2mm in thickness.

In one embodiment, the intermediate layer comprises nylon.

In one embodiment, the intermediate layer is made from a nylon.

In one embodiment, the intermediate layer is made from a material comprising a nylon.

In one embodiment, the intermediate layer comprises 19.6% nylon or about 19.6% nylon.

In one embodiment, the intermediate layer is made from a material comprising 19.6% nylon or about 19.6 % nylon.

In one embodiment, the intermediate layer comprises 74.4% polyester, 19.6% polyamide (nylon) and 6% elastane.

In one embodiment, the intermediate layer is 2mm or about 2mm in thickness.

In one embodiment, the intermediate layer is 1.3 mm or about 1.3 mm in thickness.

In one embodiment, the textile assembly is adapted to retain liquid when external force or pressure is applied from a direction that is orthogonal or substantially orthogonal to the surface area the textile assembly.

In one embodiment, the textile assembly is adapted to retain liquid unless the textile assembly is twisted or wrung or squeezed. In other words, the textile assembly is adapted to release liquid or only release liquid when the textile assembly is twisted, wrung or squeezed.

In one embodiment, the textile assembly is adapted to retain more liquid when the textile assembly is unstressed and adapted to retain less liquid when stress is applied to said assembly (such as experienced during twisting or squeezing of textile assembly).

In one embodiment, the first second and intermediate layers are contiguous each other.

In one embodiment, at least one layer of a material may interpose between at least one of the first and intermediate layer and the second and intermediate layer.

In one embodiment, the textile assembly comprises a leakproof layer sealing and/or trapping liquid at the second layer at least on a side of the second layer that is opposite to side of the second layer that is proximal to the intermediate layer.

In one embodiment, the leakproof layer is laminated, stitched, attached or otherwise adhered to at least the second layer.

In one embodiment, the leakproof layer is resin coated woven leakproof textile.

In one embodiment, the leakproof layer comprises or is made from 100% polyamide.

In one embodiment, the textile assembly further comprises a fourth layer located between the first layer and the intermediate layer.

In one embodiment, the first layer and the intermediate layer are laminated, stitched, attached or otherwise adhered to at least the fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the fourth layer is formed as a carbon crystal mesh.

In one embodiment, the textile assembly is able to or is incorporated with or adapted for use as part of or is a garment.

Further disclosed herein is a multi-layer textile assembly for absorbing and retaining liquid emitted from a human body, the textile assembly comprising:
a first layer made of or constructed as a pique knitted, one-way wicking material,
a second layer made of or constructed as a microfibre material, and
an intermediate layer made of or constructed as a warp knitted spacer fabric,
the intermediate layer being positioned between the first layer and the second layer and is adapted to interconnect the first layer and the second layer,
wherein, the first layer is adapted to transfer liquid from a first side of the textile assembly to the intermediate layer, the intermediate layer is adapted to receive the liquid from the first layer and the second layer is adapted to retain the liquid at or proximal to a second side of the textile assembly that is oppositely facing to the first side.

In one embodiment the intermediate layer is made of or constructed as the warp knitted spacer fabric having a plurality of sections with at least one of the plurality of sections being proximal to the first layer and at least another one of the plurality of sections being proximal to the second layer.

In one embodiment the sections are of a multi-layer assembly.

In one embodiment, at least one and preferably each of the plurality of sections has a knitting density that is different from a knitting density of another section or sections.

In one embodiment the sections are of a multi-layer assembly.

In one embodiment, the plurality of sections comprise at least three sections that are a first section, a second section and a middle section with the middle section interconnecting the first and second sections, the first section being proximal to the first layer and the second section being proximal to the second layer, wherein the knitting density of the first, second and middle sections are high to low in the following order:
i. the second section
ii. the first section
iii. the middle section.

In one embodiment, the liquid transferred from the first layer is adapted to be received by the first section, middle section and the second section of the intermediate layer in the following sequential order before being transferred to the second layer:
i. the first section
ii. the middle section
iii. the second section.

In one embodiment, the first section is warp knitted to form a plurality of pores to receive the liquid from the first layer.

In one embodiment, the second section is knitted in a one-way knitting pattern to help prevent liquid in the second layer from flowing back to the intermediate layer.

In one embodiment, the middle section is warp knitted to form substantially vertically oriented fibres.

In one embodiment, the second section is warp knitted to form a plurality of pores to receive the liquid from the middle section, wherein pores of the second section are smaller in size than the pores of the first section.

In one embodiment, when the textile assembly is twisted, wrung or squeezed, the substantially vertically oriented fibres (or tension in the substantially vertically oriented fibres) causes at least a portion of the second section to be pulled thereby causing at least some of the pores of the second section to be wider and/or knitting density of the second section to be distorted for at least some of the liquid to escape from the textile assembly.

In one embodiment, the first layer comprises or is made from at least one hydrophobic fibre material.

In one embodiment, the microfibre material comprises a plurality of gaps in between for collecting liquid.

In one embodiment, the intermediate layer is directly or indirectly affixed to the first layer and the second layer thereby interconnecting the first layer and the second layer.

In one embodiment, the textile assembly is antibacterial and/or hypoallergenic.

In one embodiment, the textile assembly is between 4mm to 6mm in thickness.

In one embodiment, the textile assembly is stretchable (preferably two or four way stretchable).

In one embodiment, the first layer and the second layer are laminated, stitched, attached or otherwise adhered to at least the intermediate layer.

In one embodiment, the first layer, the second layer and the intermediate layer are assembled to each other using adhesive.

In one embodiment, the first, second and intermediate layers are co-extensive to each other.

In one embodiment, the adhesive comprises spacing to allow the textile assembly to have wicking properties.

In one embodiment, the weight of the textile assembly is or about 410 grams per square meter (gsm).

In one embodiment at least one (and preferably all) of the first layer, the second layer and the intermediate layer have hypoallergenic properties.

In one embodiment, the first layer is formed as a pique knitted mesh.

In one embodiment, the first layer is antibacterial.

In one embodiment, the first layer is odour resistant.

In one embodiment, the first layer has a (fast) wicking and drying properties.

In one embodiment, the first layer comprises a polyester.

In one embodiment, the first layer comprises polyamide, polyester or a combination thereof.

In one embodiment, the first layer comprises only a polyester.

In one embodiment, the first layer is made from a material comprising a polyester.

In one embodiment, the first layer comprises 37.2% or about 37.2% polyester or is made from a material comprising 37.2% or about 37.2 % polyester.

In one embodiment, the first layer is made from a material that comprises spandex/elastane.

In one embodiment, the first layer is made from a material comprising spandex/elastane.

In one embodiment, the first layer comprises 6% spandex/elastane or is made from a material comprising 6% spandex/elastane.

In one embodiment, the weight of the first layer is 170 grams per square meter (gsm).

In one embodiment, the microfibre material is a stretch microfibre material.

In one embodiment, the second layer comprises a highly absorbent and liquid retaining microfibre towelling or is made from a highly absorbent and liquid retaining microfibre towelling.

In one embodiment, the second layer comprises a polyester.

In one embodiment, the second layer comprises only a polyester.

In one embodiment, the second layer is made from a material comprising a polyester.

In one embodiment, the second layer is made from a material comprising 37.2% polyester or about 37.2% polyester.

In one embodiment, the second layer comprises 37.2% polyester or about 37.2% polyester.

In one embodiment, the second layer is made from a material comprising spandex/elastane.

In one embodiment, the second layer material comprises spandex/elastane.

In one embodiment, the second layer is made from a material comprising 6% spandex/elastane.

In one embodiment, the second layer comprises 6% spandex/elastane.

In one embodiment, the second layer comprises a polyamide.

In one embodiment, the second layer is made from a material comprising a polyamide.

In one embodiment, the second layer is made from a material comprising 12% or about 12% spandex/elastane.

In one embodiment, the second layer comprises 12% or about 12% spandex/elastane.

In one embodiment, the second layer is made from a material comprising 12% or about 12% polyamide.

In one embodiment, the second layer comprises 12% or about 12% polyamide.

In one embodiment, the second layer is made from a material comprising 76% or about 76% polyester.

In one embodiment, the second layer comprises 76% or about 76% polyester.

In one embodiment the second layer comprises 76% Polyester, 12% polyamine and 12% elastane.

In one embodiment, the microfibre material comprises a liquid resistant oil print to retain liquids.

In one embodiment, the microfibre material is 2mm or about 2mm in thickness.

In one embodiment, the intermediate layer is made from a nylon.

In one embodiment, the intermediate layer comprises nylon.

In one embodiment, the intermediate layer is made from a material comprising a nylon.

In one embodiment, the intermediate layer is made from a material comprising 19.6% nylon or about 19.6% nylon.

In one embodiment, the intermediate layer comprises 19.6% nylon or about 19.6% nylon.

In one embodiment, the intermediate layer comprises 74.4% polyester, 19.6% polyamide (nylon) and 6% elastane.

In one embodiment, the intermediate layer is 2mm or about 2mm in thickness.

In one embodiment, the intermediate layer is 1.3 mm or about 1.3 mm in thickness.

In one embodiment, the textile assembly is adapted to retain liquid when external force or pressure is applied from a direction that is orthogonal or substantially orthogonal to the surface area the textile assembly.

In one embodiment, the textile assembly is adapted to retain liquid unless the textile assembly is twisted, wrung or squeezed. In other words, the textile assembly is adapted to release liquid or only release liquid when the textile assembly is twisted wrung or squeezed.

In one embodiment, the textile assembly is adapted to retain more liquid when the assembly is unstressed and adapted to retain less liquid when stress is applied to said assembly (such as experienced during twisting or squeezing of the textile assembly).

In one embodiment, the first second and intermediate layers are contiguous to each other.

In one embodiment, at least one layer of a material may interpose between at least one of the first and intermediate layer and the second and intermediate layer.

In one embodiment, the textile assembly comprises a waterproof layer sealing and/or trapping liquid at the second layer at least on a side of the second layer that is opposite to side of the second layer that is proximal to the intermediate layer.

In one embodiment, the waterproof layer is laminated, stitched, attached or otherwise adhered to at least the second layer.

In one embodiment, the waterproof layer is resin coated woven waterproof textile.

In one embodiment, the waterproof layer comprises or is made from 100% polyamide.

In one embodiment, the textile assembly further comprises a fourth layer located between the first layer and the intermediate layer.

In one embodiment, the first layer and the intermediate layer are laminated, stitched, attached or otherwise adhered to at least the fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the fourth layer is formed as a carbon crystal mesh.

Preferably the textile assembly is able to or is incorporated with or adapted for use as part of or is a garment.

In a second aspect, the invention resides in a method of making a multi-layer liquid absorbing and retaining textile assembly (textile fabric) for absorbing and retaining liquid emitted from a human body, as defined in the first aspect, the method comprising:
providing a first layer made of or constructed as a pique-knitted one-way wicking material,
providing a second layer that is tricot knitted and made of or constructed as a microfibre material,
providing an intermediate layer that is made of or constructed as warp knitted spacer fabric having a plurality of sections with at least one of the plurality of sections being configured to be proximal to the first layer and at least another one of the plurality of sections being proximal to the second layer,
positioning the intermediate layer between the first layer and the second layer and directly or indirectly affixing the intermediate layer to the first layer and the second layer so that the intermediate layer interconnects the first layer and the second layer.

Providing an intermediate layer made of or constructed as a warp knitted spacer fabric comprises:
warp knitting the intermediate layer in at least three sections that are a first section, a second section and a middle section with the middle section interconnecting the first and second sections.

Positioning the intermediate layer between the first layer and the second layer comprises:
positioning the first section proximal to the first layer and the second section being proximal to the second layer.

The method comprises warp knitting the first section to form a plurality of pores to receive the liquid from the first layer.

The method comprises warp knitting the middle section to form substantially vertically oriented fibres.

The method comprises warp knitting the second section to form a plurality of pores to receive the liquid from the middle section, wherein size of the pores of the second section are smaller than the size of the pores of the first section.

The method further comprises warp knitting the first, second and middle sections to have a knitting density from high to low in the following order:
i. the second section
ii. the first section
iii. the middle section.

The method further comprises positioning the intermediate layer between the first layer second layer thereby interconnecting the first layer and the second layer so that the first layer is able to transfer liquid from a first side of the textile assembly to the intermediate layer, the intermediate layer is able to receive the liquid from the first layer and the second layer is able to retain the liquid at or proximal to a second side of the textile assembly that is oppositely facing to the first side.

In one embodiment, the method further comprises laminating, stitching, attaching or otherwise adhering the first layer and the second layer to the intermediate layer to interconnect the intermediate layer to the first and second layers.

In one embodiment, the method further comprises providing a leakproof layer and sealing the second layer by the leakproof layer at least on a side of the second layer that is opposite to side of the second layer that proximal to the intermediate layer.

In one embodiment, the method further comprises laminating, stitching, attaching or otherwise adhering the leakproof layer to at least the second layer.

In one embodiment, the leakproof layer is resin coated woven leakproof textile.

In one embodiment, the leakproof layer comprises or is made from 100% polyamide.

In one embodiment, the textile assembly further comprises a fourth layer located between the first layer and the intermediate layer.

In one embodiment, the first layer and the intermediate layer are laminated, stitched, attached or otherwise adhered to at least the fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the fourth layer is formed as a carbon crystal mesh.

In one embodiment, the multi-layer liquid absorbing and retaining textile assembly is the one as defined in any one of the above statements.

Also disclosed herein is a method of making a multi-layer liquid absorbing and retaining textile assembly (textile fabric) for absorbing and retaining liquid emitted from a human body, the textile assembly comprising:
providing a first layer of a pique knitted, one-way wicking configuration,
providing a second layer that is tricot knitted and liquid transferring and retaining configuration,
providing an intermediate layer made of or constructed as a warp knitted spacer fabric having a plurality of sections with at least one of the plurality of sections being configured to be proximal to the first layer and at least another one of the plurality of sections being proximal to the second layer,
positioning the intermediate layer between the first layer and the second layer and directly or indirectly affixing the intermediate layer to the first layer and the second layer so that the intermediate layer interconnects the first layer and the second layer.

In one embodiment, providing an intermediate layer made of or constructed as a warp knitted spacer fabric comprises:
warp knitting the intermediate layer in at least three sections that are a first section, a second section and a middle section with the middle section interconnecting the first and second sections.

In one embodiment, positioning the intermediate layer between the first layer and the second layer comprises:
positioning the first section proximal to the first layer and the second section being proximal to the second layer.

In one embodiment, the method comprises warp knitting the first section to form a plurality of pores to receive the liquid from the first layer.

In one embodiment, the method comprises warp knitting the middle section to form substantially vertically oriented fibres.

In one embodiment, the method comprises warp knitting the second section to form a plurality of pores to receive the liquid from the middle section, wherein size of the pores of the second section are smaller than the size of the pores of the first section.

In one embodiment, the method further comprises warp knitting the first, second and middle sections to have a knitting density from high to low in the following order:
i. the second section
ii. the first section
iii. the middle section.

In one embodiment, method further comprises positioning the intermediate layer between the first layer second layer thereby interconnecting the first layer and the second layer so that the first layer is able to transfer liquid from a first side of the textile assembly to the intermediate layer, the intermediate layer is able to receive the liquid from the first layer and the second layer is able to retain the liquid at or proximal to a second side of the textile assembly that is oppositely facing to the first side.

In one embodiment, the method further comprises laminating, stitching, attaching or otherwise adhering the first layer and the second layer to the intermediate layer to interconnect the intermediate layer to the first and second layers.

In one embodiment, the leakproof layer is resin coated woven leakproof textile.

In one embodiment, the leakproof layer comprises or is made from 100% polyamide.

In one embodiment, the method further comprises providing a leakproof layer and sealing the second layer by the leakproof layer at least on a side of the second layer that is opposite to side of the second layer that proximal to the intermediate layer.

In one embodiment, the method further comprises laminating, stitching, attaching or otherwise adhering the leakproof layer to at least the second layer.

In one embodiment, the leakproof layer is resin coated woven leakproof textile.

In one embodiment, the leakproof layer comprises or is made from 100% polyamide.

In one embodiment, the textile assembly further comprises a fourth layer located between the first layer and the intermediate layer.

In one embodiment, the first layer and the intermediate layer are laminated, stitched, attached or otherwise adhered to at least the fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the fourth layer is formed as a carbon crystal mesh.

In one embodiment, the multi-layer liquid absorbing and retaining textile assembly is the one as defined in any one of the above statements.

Further disclosed is a method of making a multi-layer liquid absorbing and retaining textile assembly (herein also referred to as "textile fabric") for absorbing and retaining liquid emitted from a body, the textile assembly comprising:
providing a first layer made of or constructed as a pique knitted, one-way wicking material,
providing a second layer made of or constructed as a microfibre material,
providing an intermediate layer made of or constructed as a warp knitted spacer fabric having a plurality of sections with at least one of the plurality of sections being configured to be proximal to the first layer and at least another one of the plurality of sections being proximal to the second layer,
positioning the intermediate layer between the first layer and the second layer and directly or indirectly affixing the intermediate layer to the first layer and the second layer so that the intermediate layer interconnects the first layer and the second layer.

In one embodiment, providing an intermediate layer made of or constructed as a warp knitted spacer fabric comprises:
warp knitting the plurality of sections with at least one of the plurality of sections being proximal to the first layer and at least another one of the plurality of sections being proximal to the second layer.

In one embodiment, providing an intermediate layer made of or constructed as a warp knitted spacer fabric comprises:
warp knitting the intermediate layer in at least three sections that are a first section, a second section and a middle section with the middle section interconnecting the first and second sections.

In one embodiment, positioning the intermediate layer between the first layer and the second layer comprises:
positioning the first section proximal to the first layer and the second section being proximal to the second layer.

In one embodiment, the method comprises warp knitting the first section to form a plurality of pores to receive the liquid from the first layer.

In one embodiment, the method comprises warp knitting the middle section to form substantially vertically oriented fibres.

In one embodiment, the method comprises warp knitting the second section to form a plurality of pores to receive the liquid from the middle section, wherein size of the pores of the second section are smaller than the size of the pores of the first section.

In one embodiment, the method further comprises warp knitting the first, second and middle sections to have a knitting density from high to low in the following order:
i. the second section
ii. the first section
iii. the middle section.

In one embodiment, method further comprises positioning the intermediate layer between the first layer second layer thereby interconnecting the first layer and the second layer so that the first layer is able to transfer liquid from a first side of the textile assembly to the intermediate layer, the intermediate layer is able to receive the liquid from the first layer and the second layer is able to retain the liquid at or proximal to a second side of the textile assembly that is oppositely facing to the first side.

In one embodiment, the method further comprises laminating, stitching, attaching or otherwise adhering the first layer and the second layer to the intermediate layer to interconnect the intermediate layer to the first and second layers.

In one embodiment, the method further comprises providing a leakproof layer and sealing the second layer by the leakproof layer at least on a side of the second layer that is opposite to side of the second layer that proximal to the intermediate layer.

In one embodiment, the method further comprises laminating, stitching, attaching or otherwise adhering the leakproof layer to at least the second layer.

In one embodiment, the method comprises coating the leakproof layer with resin to form resin coated woven leakproof textile.

In one embodiment, the leakproof layer comprises or is made from 100% polyamide.

In one embodiment, the method further comprises laminating, stitching, attaching or otherwise adhering the first and second layers to at least a fourth layer.

In one embodiment, the fourth layer is a light knit textile material comprising carbon powder or crystals.

In one embodiment, the fourth layer comprises or is made of polyester with carbon crystals or powder embodied in the fourth layer.

In one embodiment, the fourth layer comprises or is made of polyester comprising carbon.

In one embodiment, the fourth layer comprises or is made of polyester with carbon crystals or powder embodied in the fourth layer.

In one embodiment, the multi-layer liquid absorbing and retaining textile assembly is the one as defined in any one of the above statements.

In an eighth aspect, the invention resides in an article of clothing comprising or that is made at least in part of or manufactured from any one or more of the textile assembly (textile fabric) as defined in any one or more of the above statements.

In one embodiment, the article of clothing is an underwear crotch.

In one embodiment, the fourth layer is formed as a carbon crystal mesh.

For purposes of the description hereinafter, the terms "upper", "lower", "right", "left", "vertical", "horizontal", "top", "bottom", "lateral", "longitudinal" and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following description are simply exemplary embodiments of the invention. Hence, specific dimensions and other physical characteristics related to the embodiments disclosed herein are not to be considered as limiting.

It is acknowledged that the term 'comprise' may, under varying jurisdictions, be attributed with either an exclusive or an inclusive meaning. For the purpose of this specification, and unless otherwise noted, the term 'comprise' shall have an inclusive meaning-i.e. that it will be taken to mean an inclusion of not only the listed components it directly references, but also other non-specified components or elements. This rationale will also be used when the term 'comprises' or 'comprised' or 'comprising' is used in relation to the apparatus or to one or more steps in a method or process.

As used hereinbefore and hereinafter, the term "and/or" means "and" or "or", or both.

As used hereinbefore and hereinafter, "(s)" following a noun means the plural and/or singular forms of the noun.

As used hereinbefore and hereinafter and unless stated otherwise, the word `liquid' in the context of this specification may include `liquid' but may equally include 'moisture'.

As used hereinbefore and hereinafter and unless stated otherwise, the word `retain' in the context of this specification may mean `continue to hold or contain something' which is different from `absorb' which in the context of the specification may mean 'to take in a liquid from the surface or space around'. Unless otherwise state, this rationale will also apply for the word(s) `retaining' and/or 'retention' as used hereinbefore and hereinafter.

When used in claim and unless stated otherwise, the word 'for' is to be interpreted to mean only `suitable for', and not for example, specifically 'adapted' or 'configured' for the purpose that is stated.

It is known, to those with skill in the art of patenting, that the word 'substantially' can, in some instances, be used to broaden a term. It should be stated that, in the present application, use of the word 'substantially' with a term, to define a (characterizing) feature(s), gets all the benefit (i.e the benefit of any broadening) afforded by use of the word 'substantially', and also includes within its scope the feature(s) being that term exactly, (without broadening). For example, if a feature is described/defined in the present application as being 'substantially vertical', then that includes, within its scope, the features being 'close' to vertical (in so far as the word 'substantially' is deemed to broaden the term 'vertical'), and also includes within its scope the features being 'exactly' vertical.

### BRIEF DESCRIPTION OF THE DRAWINGS

The invention will now be described by way of example only and with reference to the drawings in which:
**Figure 1** is a schematic cross section illustration of a piece or section of a textile assembly (textile fabric) according to a first preferred embodiment of the present invention.
**Figure 2** shows an example of a pique mesh that can be used to form one of the layers of the textile assembly of Figure 1.
**Figure 3** shows an example of a microfibre material that can be used to form one of the layers of the textile assembly of Figure 1.
**Figure 4** shows a schematic cross section illustration of the textile assembly of Figure 1 with optional leakproof layer.
**Figure 5** shows a schematic cross section illustration of the textile assembly of Figure 4 with optional fourth layer.
**Figure 6** is a schematic cross section illustration of a piece or section of a textile assembly (textile fabric) according to a second preferred embodiment of the present invention.
**Figures 7A** is a schematic illustration of a surface of a microfibre material for use in construction of a second layer of the textile assembly of Figure 6.
**Figures 7B** is a schematic illustration of a surface of a cotton
**Figure 8** illustrates a low knitting density section (a first section), an open space section (a middle section) and the high density knit section (a second section) of an intermediate layer of the textile assembly of Figure 6.
**Figure 9** illustrates a knitting pattern of a first layer of the textile assembly of Figure 6.
**Figure 10** illustrates a knitting pattern of a second layer of the textile assembly of Figure 6.
**Figure 11** illustrates an example of an underwear crotch that is made using a textile assembly according to a third preferred embodiment of the present invention.
**Figure 12** shows a schematic cross section illustration of a textile assembly of the third preferred embodiment of the present invention along axis X-X of Figure 11.
**Figure 13** shows a liquid flow and retention in the textile assembly of Figure 12.
**Figure 14** shows a direct vertical pressure being applied to the textile assembly of Figure 12.
**Figure 15** shows an example of liquid being released when the textile assembly of Figure 12 is twisted.

### DETAILED DESCRIPTION

In accordance with an exemplary embodiment of the present invention, a multi-layer textile assembly (herein also referred to as "textile fabric") is provided. This assembly may be an absorbent, washable and hydrophilic fabric that may additionally optionally also be printable, disposable, non-fraying and/or dimensionally stable. The fabric material may be highly absorbent and has a quick wicking and moisture retention capability.

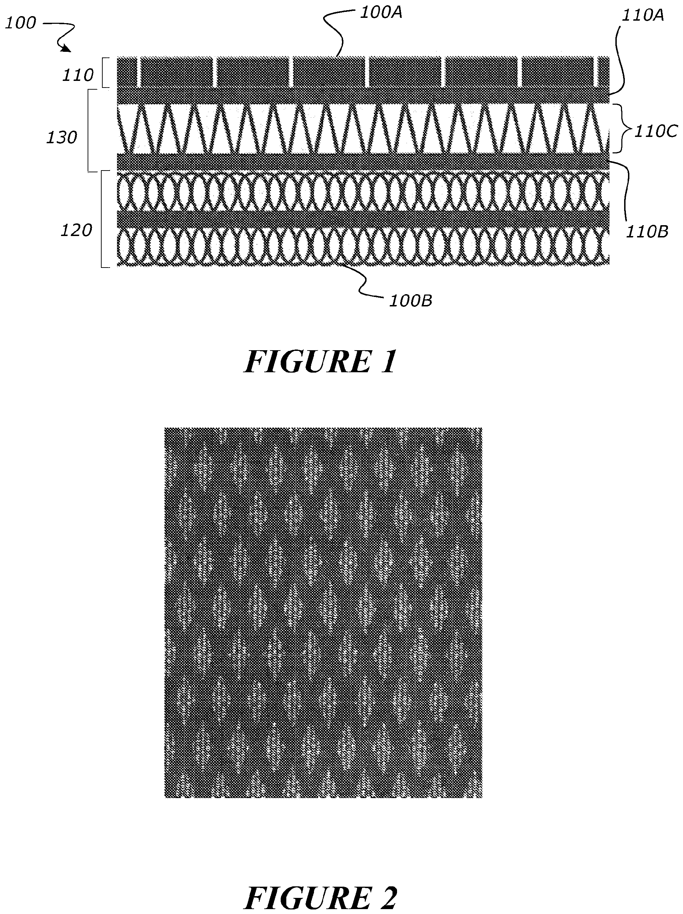

Reference will now be made to the accompanying drawings in which Figure 1 shows a schematic cross section illustration of a piece or section of a multi-layer textile assembly 100 according to a preferred embodiment of the present invention. The textile assembly 100 may be suitable for absorbing and retaining liquid- the liquid being emitted from a body of human or non-human animals. The liquid may be in the form of sweat, saliva, urine for example. The textile assembly 100 may also be suitable for absorbing and retaining liquids other than the liquids emitted from the body of human or non-human animals. Unless stated otherwise, liquid(s) in the context of this specification can be moisture(s).

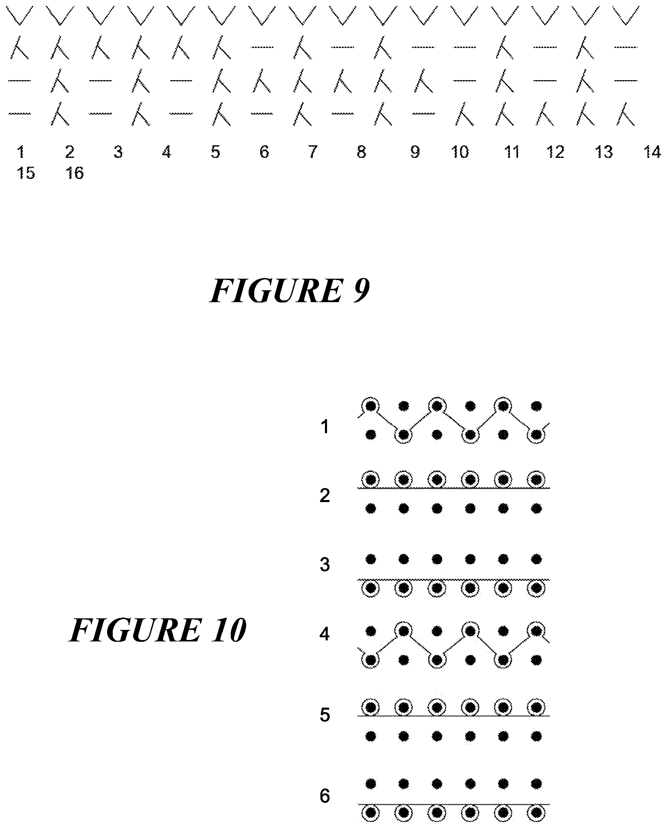

The textile assembly may comprise a first layer 110, a second layer 120 and intermediate layer 130 placed, positioned or sandwiched between the first layer 110 and the second layer 120 thereby interconnecting the first layer 110 and the second layer 120. For example, in one embodiment, the intermediate layer is 130 is directly or indirectly affixed to the first layer 110 and the second layer 120 thereby interconnecting the first layer 110 and the second layer 120. A layer of layers of other material may be interposed between the intermediate layer and one of each of the first and second layers but preferable these 3 layers are directly attached to each other by means herein described. In one embodiment, the first, second and intermediate layers are contiguous to each other. The first layer 110 may be made of or constructed as a one-way wicking fibre material. By wicking it is for example meant that the first layer 110 may be made from a material that can draw liquid from its source such as a surface which, in the context of the present invention, may be the body such as from the skin of a person. Wicking may be due to capillary action or it may be a form of absorbency. Wicking may be due to hydrophilic properties of the first layer which can be obtained for example by constructing the first layer to have an increased surface area or by constructing the first layer using a knitting pattern such as the one as shown in Figure 9. Similarly, gravity and structure of the textile assembly with the first layer 110 having open pores to absorb and transfer liquid from top to the second layer 120 at the bottom may mean that the textile assembly 100 is denser on the second layer 120 thereby facilitating one-way wicking.

The second layer 120 may be made of is constricted as a material that retains liquid(s), for example, the liquid(s) emitted from a human body.

The intermediate layer 130 may be made of or constructed as a warp knitted spacer fabric. The intermediate layer 130 may be hydrophilic and therefore may be adapted to absorb and receive the liquid from the first layer 110. As shown in Figure 1, the intermediate layer 130 may be made of or constructed as a warp knitted spacer fabric having a plurality of sections (that are layered) with at least one of the plurality of sections being proximal to the first layer 110 and at least another one of the plurality of sections being proximal to the second layer. Having a plurality of sections in such configuration is advantageous as it allows a controlled transfer of liquid between the first layer 110 and the second layer 120. This is because since the liquid has to transfer through multiple sections/layers of the intermediate layer before it can reach the second layer 120, the volume and speed of transfer of the liquid will be slower and therefore hence leakage can be prevented.

Although, the plurality of sections may comprise two sections, most preferably the plurality of sections comprises at least three sections, namely a first section 130A, a second section 130B and a middle section 130C interconnecting the first and the second sections 130C as shown in Figure 1. The first section 130A is proximal to the first layer 110 and the second section 130B is proximal to the second layer 120. As shown, the first section 130A is the top/upper section, the second section 130B is the bottom/lower section and the middle section 130C is the middle/intermediate section disposed between the first and second sections. As shown, the first section 130A, the second section 130B and the middle section 130C are each formed as a layer.

The knitting density each of the sections 130A, 130B and 130C may also be different. For example, as it can be seen from Figure 1, middle section 130C is more open than the first section 130A and the second section 130B, which means that the middle section will have the lower knitting density than the knitting density of the first section 130A and the knitting density of the second section 130B. Preferably, the knitting density of the second section 130B may be higher than that of the first section 130A. Knitting density may mean having a same knit but having more knots per unit area (e.g. more knots per meter square centimetre) and consequently the higher the knitting density, the higher may be the weight per unit area (per square centimetre) of fabric. Having such plurality of sections with different knitting density from one another can be advantageous and can further enhance a controlled transfer of liquid between the first layer 110 and the second layer 120. This is because, the higher the knitting density of a section lesser will be the rate of liquid that can pass through that section. In one example embodiment, during use, the first layer 110 (more specifically first face side 100A of textile assembly 100) may be in contact with a surface e.g. to a skin of a person who is wearing an article of clothing containing or made out of the textile assembly 100. The first layer 110 may absorb the liquid from the body of the person via the first face side 110A (first side) and transfer the liquid to the intermediate layer 130. One-way wicking properties of the first layer 110 may mean the liquid may only be transferred from the first layer 110 to intermediate layer 120 and not vice versa. The intermediate layer 130 may receive the liquid from the first layer (110) and the second layer retain the liquid so that the liquid does not easily escape from the second face side 100B (second side) of the textile assembly that is opposite to the first face side. The first face side 100A may be the top of the textile assembly and the second face side 100B may be the bottom of the textile assembly 100.

The textile assembly 100 may preferably be between 4mm to 6mm in thickness, although may be less than 4mm thick or more than 6mm thick.

The textile assembly 100 may be stretchable (preferably two or four way stretchable). In one preferred example of the embodiment, the ratio of the stretchability of the most stretchable condition to the least stretchable condition of the textile assembly 100 may be up to 1.3.

The first layer 110 and the second layer 120 may be laminated, attached or otherwise adhered to at least the intermediate layer 130 to interconnect the intermediate layer 130 to the first layer 110 and second layer 120. For example, the first layer 110 and the second layer 120 may be stitched to at least the intermediate layer 130. Alternatively, or additionally, the first layer 110, the second layer 120 and the intermediate layer 130 may be assembled or bonded together using adhesive, thereby forming a single layer or integrated/composite fabric.

In an embodiment where the adhesive is used to assemble or bond the first layer 110, the second layer 120 and the intermediate layer 130, the adhesive may comprise spacing (or gaps) to allow the textile assembly 100 to have/ maintain the wicking properties. In other words, the adhesive may form an adhesive layer having spacing or gap(s) to allow the textile assembly to have/maintain the wicking properties. The adhesive may be screen printed on a layer(s) of the textile assembly with a mesh type pattern that creates gaps between the adhesives. Pattern may vary based on needs. Adhesives may also be spot applied hence creating gaps between adhesive zones.

The weight of the textile assembly may optionally be or about 410 grams per square meter (gsm).

The first layer 110, the second layer 120 and/or the intermediate layer 130 may be hypoallergenic and/or antibacterial or may have hypoallergenic and/or antibacterial properties. In one configuration, all of the three layers 110, 120, 130, i.e. the whole textile assembly 100 may be constructed from a material that does not use natural fibre thereby making the layers 110, 120, 130 or the textile assembly 100 to be hypoallergenic and/or antibacterial or to have hypoallergenic and/or antibacterial properties.

The first layer 110 may comprise or made from a polyester or from a material comprising a polyester. For example, the first layer may comprise or be may be made from a material comprising 37.2% or about 37.2 % polyester. In one configuration, the first layer 110 may comprise or be made from a material comprising polyester only (i.e. 100% polyester). In one embodiment, the first layer 110 may comprise or be made from a material comprising polyester and spandex (i.e. elastane), or polyester and nylon.

The first layer 110 may optionally be pique knitted or be of a pique knitted configuration. For example, the first layer may be formed as a pique knitted mesh or pique mesh that transfers liquid away from the body. By being pique knitted the first layer may have high strength, be breathable and be durable.

The first layer 110 or pique mesh may be antibacterial and at least for that reason may also be odour resistant.

The first layer 110 may have faster wicking and drying properties. For example, the first layer 110 may be made using polyester and/or polyamide which are hydrophobic fibres but structured in a way that creates pores to transfer liquid thereby providing faster drying and wicking properties. The first layer 110 may be breathable and may use body heat for fast drying during use/wear.

The first layer 110 may comprise or be made from a material comprising elastane for example.

The weight of the first layer may optionally be 170 grams per square meter (gsm), although it is possible that the weight is more than or less than 170 gsm.

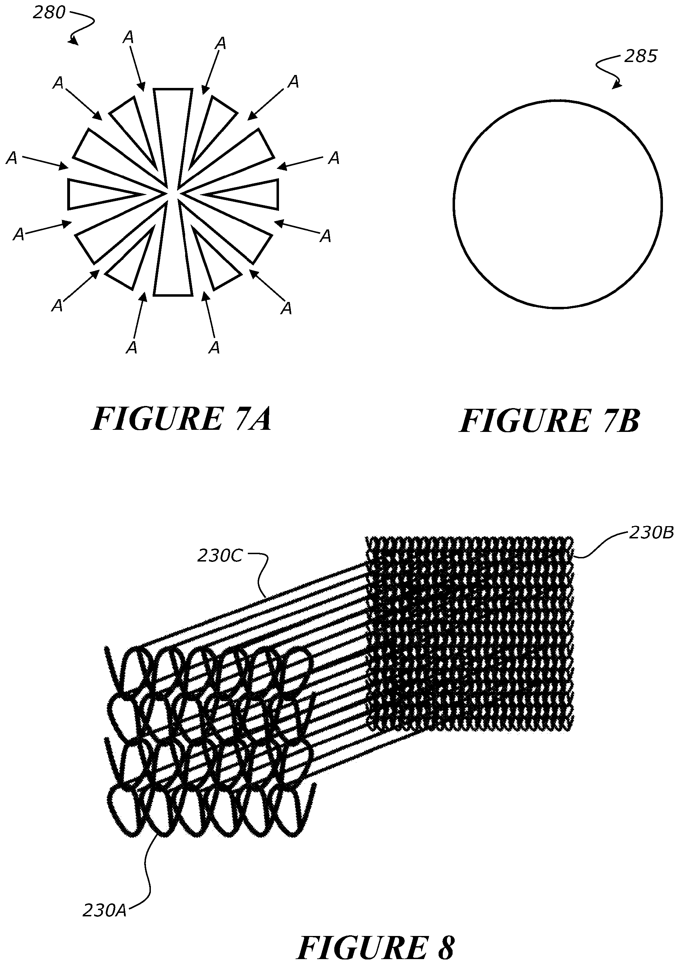

The second layer 120 is most preferably made or constructed as a material that is a microfibre such as a stretch microfibre. The microfibre or the construction material of the second layer (120) may be a highly absorbent and liquid retaining microfibre towelling. The microfibre may have a plurality of gaps in between for collecting liquid. Such gaps allow the microfibre to have higher surface area than traditional natural fibres such as cotton in order to collect large amounts of liquid and absorb the desired volume of liquid to be discharged. This is described later in more detail with reference to Figure 7A. Optionally, the second layer 120 may comprise or be made from a material comprising a polyester. For example, the second layer 120 may be made from a material comprising 37.2% polyester or about 37.2 % polyester. In one configuration, the second layer 110 may comprise or be made from a material comprising polyester only.

Optionally or additionally, the second layer may comprise or be made from a material comprising spandex (i.e. elastane) for example, 6% spandex/elastane or 12% spandex/elastane.

Optionally or additionally, the second layer 120 may comprise or be made from a polyamide or a material comprising a polyamide, for example 12% or about 12% polyamide.

Optionally or additionally, the second layer 120 may comprise or be made from a material comprising 76% or about 76% polyester. In one embodiment the second layer may comprise 76% polyester, 12% polyamine and 12% elastane.

The second layer 120 may optionally comprise a liquid resistant oil print or some other coatings of liquid resistant material in order to retain liquids. The second layer 120 may be tricot knitted or is of tricot knitted configuration. Having tricot knitted allows better liquid retention than conventional materials/natural fibres such as cotton and also allows the second layer 120 of the textile assembly 100 to hold liquid under pressure. The second layer 120 may be antibacterial, odour resistant and breathable.

The second layer 120 may be 2mm or about 2mm in thickness, although it is possible that thickness of the second layer 120 is more than or less than 2 mm.

The intermediate layer 130 may comprise or be made from a nylon or from a material comprising a nylon. For example, the intermediate layer 130 may comprise or be made from a material comprising 19.6% nylon or about 19.6 % nylon. In one embodiment, the intermediate layer 130 may comprise 74.4% polyester, 19.6% polyamide (nylon) and 6% elastane.

The intermediate layer may be 2mm or about 2mm in thickness, although it is possible that thickness of the second layer 120 is more than or less than 2mm (for example 1.3mm or about 1.3mm).

Having the intermediate layer 130 made of a warp knitted fabric can facilitate transfer of liquid to the second layer 120.

The textile assembly 100 may be adapted to retain liquid when external force or pressure is applied from a direction that is orthogonal or substantially orthogonal to the surface area the textile assembly. For example, when under pressure (compression acting parallel the normal of the face sides) from body weight any liquid that is retained within the fabric may not escape from the textile assembly 100. The liquid may be more easily able to escape or released when the textile fabric 100 is twisted, wrung, or squeezed. The second section 130B may be knitted in a one-way knitting pattern similar to the first layer 110 which helps prevent the liquid transferred to the second layer 120 from flowing or otherwise entering/flowing back to the intermediate layer 130. The knitting pattern of the first layer 110 may be similar to the one as shown in Figure 9.

The intermediate layer 130 may be constructed as warp knitted fabric having an inner spacer (middle section 130C) with substantially vertical fibers and that may keep its structure under direct vertical pressure but once twisted (e.g. during washing cycles) the structure of the intermediate layer 130 is distorted thereby temporarily opening wider pores in the fabric for liquid to escape with twist motion. The ability for the liquid to be squeezed out or escape when twisted, wrung or squeezed (for example during wash) would mean the textile assembly 100 can facilitate fast drying. When the textile assembly 100 is twisted, substantially vertical fibres of the middle section 130C will be stretched and such stretching of the substantially vertical fibres will pull at least some portion of the second section 130B thereby causing at least some of the pores of the second section 130B to be wider and/or distorting the density the knitted fibres of the second section 130B for the liquid to escape easily. In other words, when the textile assembly 100 is twisted, wrung or squeezed, the substantially vertically oriented fibres (or tension in the substantially vertically oriented fibres) causes at least a portion of the second section 130B to be pulled/stretched thereby causing at least some of the pores of the second section 130B to be wider and/or knitting density of the second section 130B to be distorted for at least some of the liquid to escape from the textile assembly 100.

Although, in Figure 1, the fibres may be oriented as even more substantially vertical fibres than what is shown in Figure 1. For example, the fibres may be oriented between the angle of 80 degrees to 110 degrees. Such substantially vertically oriented fibres can create open space as shown in Figure 1 and consequently such open space can create the airflow within the middle section 130C which can also allow faster drying of the first layer 110 one the liquid is transferred to the second layer 120.

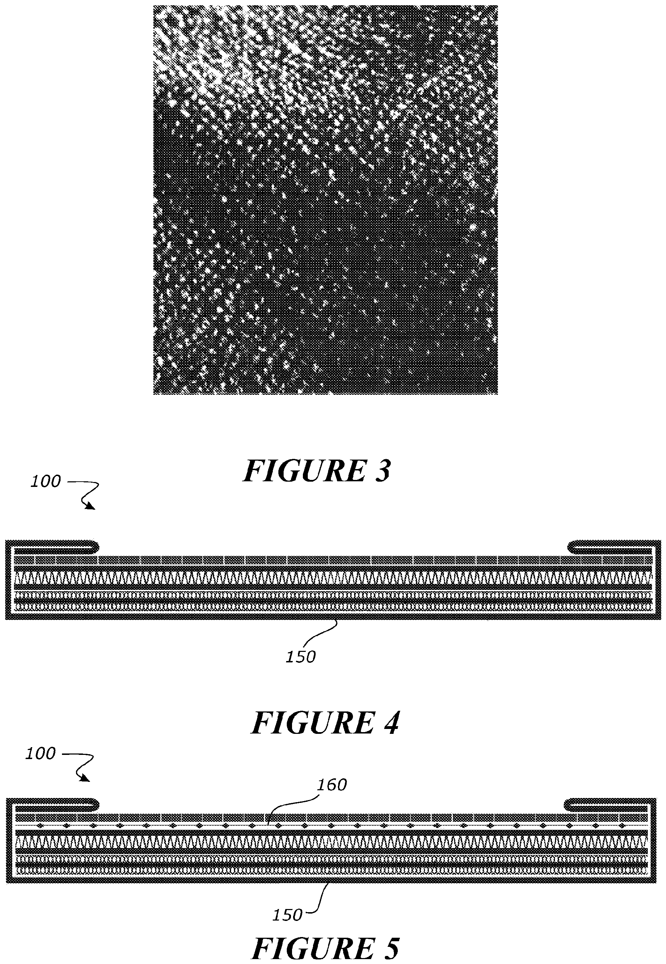

As shown in Figure 4, the textile assembly may optionally comprise a leakproof/leakproof layer 150. The leakproof layer 150 may be used to seal the second layer 120 at least on a side of the second layer 120 that is opposite to the side that is adjacent or proximate to the intermediate layer 130. Such leakproof layer may be bonded, attached or otherwise adhered to the second layer 120 via many suitable means such as but not limited to using adhesives, lamination, stitching etc. In one embodiment, the leakproof layer 150 may be resin coated woven leakproof textile.

In one embodiment, the leakproof layer 150 may comprise or may be made from 100% polyamide. The leakproof layer 150 may be antibacterial, odour resistant and breathable.

Figure 5 is a schematic cross section illustration of the textile assembly 100 of Figure 4 further comprising a fourth layer 160. Although the leakproof layer 150 is shown in Figure 5 that leakproof layer 150 may be optional. The fourth layer 160 may be located between the first layer and the intermediate layer. In one embodiment, the first layer 110 and the intermediate layer 130 are laminated, stitched, attached or otherwise adhered to at least the fourth layer 160. In one embodiment, the fourth layer 160 is a light knit textile material comprising carbon powder or crystals. For example, the fourth layer 160 may comprise or made of polyester (e.g. 100% polyester) with carbon crystals or powder embodied in the fourth layer 160. Having such fourth layer 160 comprising carbon powder or crystal can be advantageous as carbon can absorb or eliminate odour. It may be possible that the fourth layer 110 comprises carbon in many other suitable form. It may be possible that the fourth layer 160 is formed as a carbon crystal mesh.

The textile assembly 100 may be used in clothing such as but not limited to garments which may also include undergarments, sportswear etc. For example, the textile assembly 100 may be able to or is incorporated with or adapted for use as part of or is a garment. The textile assembly 100 may also be used in or as cleaning cloths, shoe inserts, shoes, sleep mats, place mats, inserts for brasseries, towels, mattress covers, feminine hygiene products, diapers, bags, sweat bands, nursing pads, underwear (e.g. male, female and children underwear), seat covers, bed sheets, maternity products and other products to absorb and retain any discharged liquids.

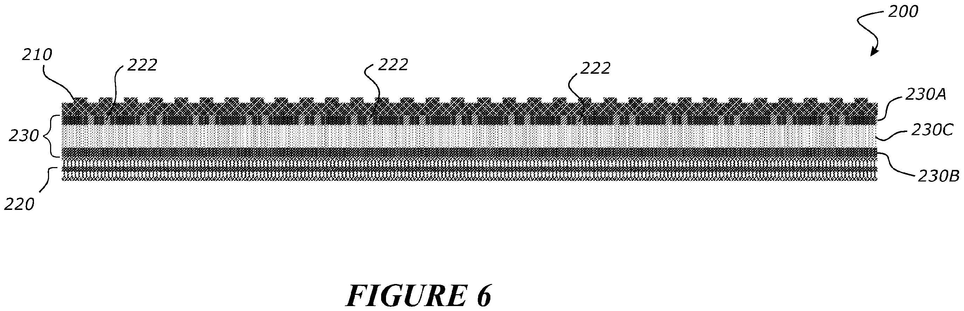

Figure 6 shows an example of a textile assembly 200 according to one example of the present invention. The textile assembly 200 of this example is similar in most aspects to the textile assembly 100 described above and the differences can be identified by comparing Figure 1 with Figure 6. In Figure 6, the features that are similar to those shown in Figures 1 are identified with the same reference numeral, incremented by 100. Most of the description of the textile assembly 100 of a preferred embodiment above, equally applies to the textile assembly 200 and therefore need not be described in detail again. Therefore, only the main features of textile assembly 200 will be discussed.

In Figure 6, the first layer 210 is a top surface (skin touching) layer that is adapted to wick away liquid discharge such as but not limited to urine, blood, sweat, breast milk from the body. The first layer 210 is made of or constructed as a pique knitted, one-way wicking material. The wicking material is preferably a hydrophobic fiber to draw liquid away from a human body. The first layer 210 may comprise or made from a polyester or from a material comprising a polyester. For example, the first layer may comprise or be may be made from a material comprising 37.2% or about 37.2% polyester. In one configuration, the first layer 110 may comprise or be made from a material comprising polyester only (i.e. 100% polyester). The first layer 210 may have faster wicking and drying properties. For example, the first layer 210 may be made using polyester which are hydrophobic fibres but structured in a way that creates pores to transfer liquid thereby providing faster drying and wicking properties. The first layer 210 may be breathable and may use body heat for fast drying during use/wear.

The second layer 220 is made or constructed as a material that is a microfibre material 280 such as a stretch microfibre material. The microfibre material 280 may have a plurality of gaps 225 in between for collecting liquid as illustrated in Figure 7A below. The microfibre material 280 may be chipped to form gaps 225, and therefore may be chipped microfibre(s). The direction of liquid transfer within the gaps 225 formed within the microfibre material 280 is shown by arrows A in the schematic diagram of Figure 7A. Such gaps 225 allow the microfibre material 280 to have higher surface area than traditional natural fibres such as cotton 285 (see Figure 7B) in order to collect large amounts of liquid and absorb the desired volume of liquid 225 to be discharged. As schematically shown in Figure 7B, natural fibres such as cotton 285 will not have such wide gaps as microfibre material 280 and therefore are not as efficient as the microfibre material 280 in collecting and retaining high volume of liquid.

The second layer 120 of the textile assembly 100 as described in the previous embodiment may also be made of or constructed of microfibre material 280 as described above.

The intermediate layer 230 is placed, positioned or sandwiched between the first layer 210 and the second layer 220 thereby interconnecting the first layer 210 and the second layer 220. The intermediate layer 230 comprises at least three sections that are a first section 230A, a second section 230B and a middle section 230C. The middle section 230C interconnects the first and second sections 230A, 230B. The first section 230A is proximal to the first layer 210 and the second section 230C is proximal to the second layer 220. The knitting density of the second section 230B may be higher than that of the first section 230A and the knitting density of the first section 230A may be higher than that of the middle section 230C. As shown, the first section 230A is the top/upper section, the second section 230B is the bottom/lower section and the middle section 230C is the middle/intermediate section that is disposed between the first and second sections. As shown, the first section 230A, the second section 230B and the middle section 230C are each formed as a layer.

During use, the liquid is transferred from the first layer 210 and is adapted to be received by the first section 230A, then the middle section 230C and then the second section 230B of the intermediate layer 220.

The first section 230A is warp knitted to form a plurality of pores 222 to receive the liquid from the first layer 210. The middle section 230C is warp knitted to form a substantially vertically oriented fibres to receive and collect a volume of liquid from the first section at a particular speed (which may be a predetermined speed). As shown, the middle section 230C of textile assembly 200 comprises more vertically oriented fibres as compared to the middle section 130C of the textile assembly 100 of the previous embodiment. By being more vertical, the middle section 230B can receive and collect a higher volume of liquid from the first section at an increased speed as compared to the middle section 130C of textile assembly 100 in which the fibres are substantially vertically oriented but is less vertically oriented than the fibres of middle section 230C of the textile assembly 200. This is because, more vertically oriented the fibres are, faster the liquid can flow due to gravity and more open is the space in which the liquid can be received and collected. As mentioned above, the second section 230B is has higher knitting density that the first and the middle sections 230A, 230C. Because, the middle section 230A has collected most liquid required at already at high speed, the second section transfers most of the liquid to the second layer 230 of the textile assembly 100 at lower speed as higher speed is no longer required. Once all the liquid is transferred to the second layer 230, the textile assembly may act as a one-way system and may not let liquid through back up even under direct body weight pressure. The textile assembly 200 will release the liquid when twisted (e.g. during washing cycles) as the density of the knitted fibres are distorted and pores are made wider for liquid to escape through. This may be in the similar manner as described above for textile assembly 100.

Approximate absorbency speed of the first layer 310 may be 3-5ml/s. Approximate absorbency speed of the intermediate layer 330 may be 5-8 ml/s. Approximate absorbency speed of the second layer 320 may be 0.5 ml/s due to slow transfer of liquid through the second section 330B of the intermediate layer 330. It will be appreciated that the absorbency rate may vary depending upon the pressure applied to the textile assembly 200.

The textile assembly 200 may be used in clothing such as but not limited to garments which may also include undergarments, sportswear etc. For example, the textile assembly 200 may be able to or is incorporated with or adapted for use as part of or is a garment. The textile assembly 200 may also be used in or as cleaning cloths, shoe inserts, shoes, sleep mats, place mats, inserts for brasseries, towels, mattress covers, feminine hygiene products, diapers, bags, sweat bands, nursing pads, underwear (e.g. male, female and children underwear), seat covers, bed sheets, maternity products and other products to absorb and retain any discharged liquids.

The textile assembly 200 may optionally comprise additional optional layers such as a leakproof layer and/or fourth layer comprising carbon powder or crystal in similar orientation as described above with reference to Figures 4 and 5.

Figure 8 illustrates the low knitting density of the first section 230A, the open space created by substantially vertically oriented fibres of middle section 230C and the higher knitting density of the second section 230B.

Figure 9 illustrates the knitting pattern of the first layer 210 of the textile assembly 200 as described above. Figure 10 illustrates the knitting pattern of the intermediate layer 230 of the textile assembly 200 as described above. The knitting patterns shown in Figures 9 and 10 will be understood by a skilled person and therefore need not be described in detail. The first layer 110 and second layer 120 of the textile assembly 100 may also be constructed with knitting patterns as shown in Figures 9 and 10 respectively.

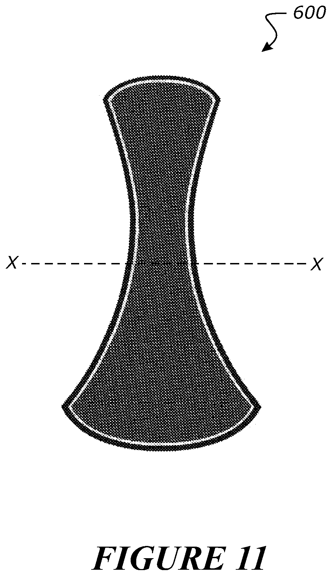

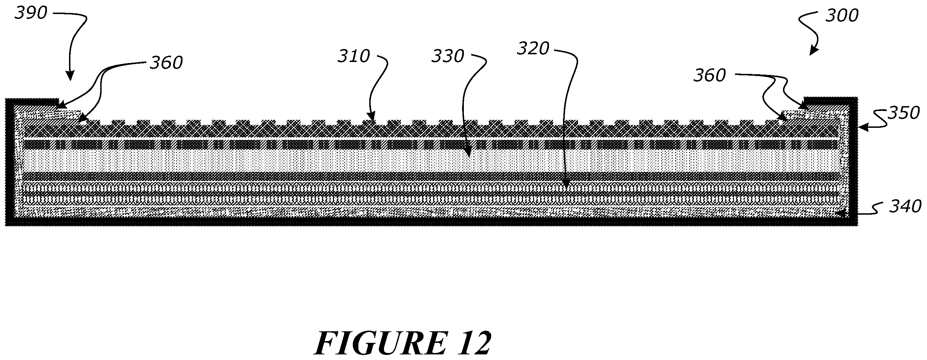

Figure 11 illustrates an example of an underwear crotch 600 that is made using a textile assembly 300 according to another preferred embodiment of the present invention. Figure 12 shows a schematic cross section illustration of the textile assembly 400 along axis X-X of Figure 11.

The textile assembly 300 is similar in most aspects to the textile assembly 200 described above and the differences can be identified by comparing Figure 6 with Figure 12. In Figure 12, the features that are similar to those shown in Figure 6 are identified with the same reference numeral, incremented by 100. Most of the description of the textile assemblies 100 and 200 of above, equally applies to the textile assembly 200 and therefore need not be described in detail again. Therefore, only the main features of textile assembly 300 will be discussed.

As shown, the textile assembly 300 comprises first layer 310, a second layer 320 and an intermediate layer 330 whose construction and orientation are identical to what is described above with reference to Figure 6 and therefore need not be described again in too much detail.

As shown in Figure 12, the textile assembly 300 may further comprise a leakproof layer 340. The leakproof layer 340 may be used to seal the second layer 340 at least on a side of the second layer 320 that is opposite to the side that is adjacent or proximate to the intermediate layer 330. Such leakproof layer 340 may be bonded, attached or otherwise adhered to the second layer 320 via many suitable means such as but not limited to using adhesives, lamination, stitching etc. In one embodiment, the leakproof layer 340 may resin coated woven leakproof textile. Preferably, the leak-proof layer 340 is made of a leakproof textile that is 100% liquid impermeable.

As shown in Figure 12, the textile assembly 300 may also comprise an outer layer 350 that may serve as design decoration and damage protection of the leakproof layer 340 for longevity of the product which in this example in an underwear crotch 600. The outer layer 350 may be used to seal the leakproof layer 340 at least on a side of the leakproof layer 340 that is opposite to the side that is adjacent or proximate to the second layer 320. The outer layer 350 may be bonded, attached or otherwise adhered to the leakproof layer 340 via many suitable means such as but not limited to using adhesives, lamination, stitching etc. Most preferably, bonding tapes 360 are used to seal the layers of the tactile assembly 300 at high temperatures together without the need to use a stich to remain substantially fully leakproof. The bonding tapes 360 may be made out of plant oil. In Figure 12, reference numeral 390 shown an extension of a leakproof layer 340 to prevent any liquid transfer on to the outer layer 350.

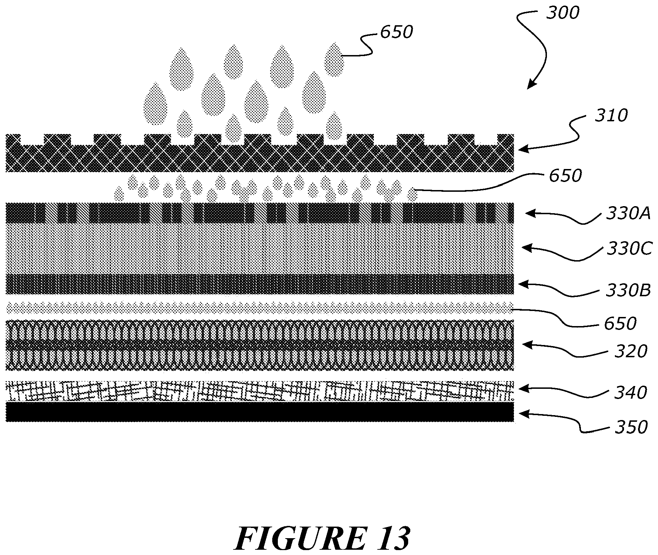

It will be appreciated that the textile assemblies 100 and 200 of the previous embodiments may also comprise optional leakproof layer and/or outer layer in similar manner and configuration as shown in Figure 13.

Figure 13 illustrates shows a liquid flow and retention in the textile assembly of Figure 12.

As shown in Figure 13, the first layer 310 receives liquid 650 and transfers the liquid to the first section 330A of the intermediate layer 330. The middle section 330C the collects and disperses the liquid 650 and transfers the liquid 650 to the second section 330B. Once most or full volume of liquid is transferred from the middle section 330C to the second layer 320, the airflow within the middle section 330B will also allow faster drying of the first layer 310.

Since the second section 330B is of high knitting density as compared to the first and middle sections 330A, 330C, the section 330B transfers liquid to second layer 320 at lower speed than the first and second sections 330A, 330C. The second section 330C works as one-way liquid transfer even with direct vertical body weight pressure, which prevents the liquid transferred to the second layer 320 from entering back to the intermediate layer 330. For example, the second section 330B may be knitted in a one-way knitting pattern similar to the first layer 310 (which in shown in Figure 9) which helps prevents liquid transferred to the second layer 320 from entering back to the intermediate layer 330.

The second layer 320 collects all or at least most of the liquid and retains the liquid within the second layer 320.

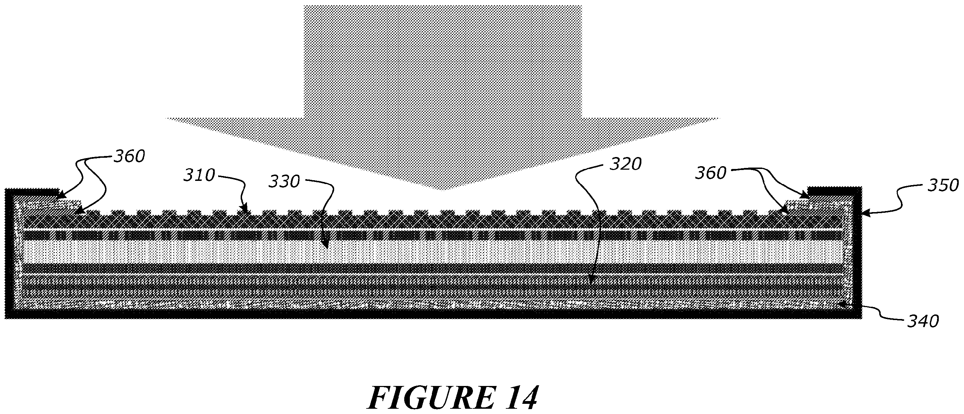

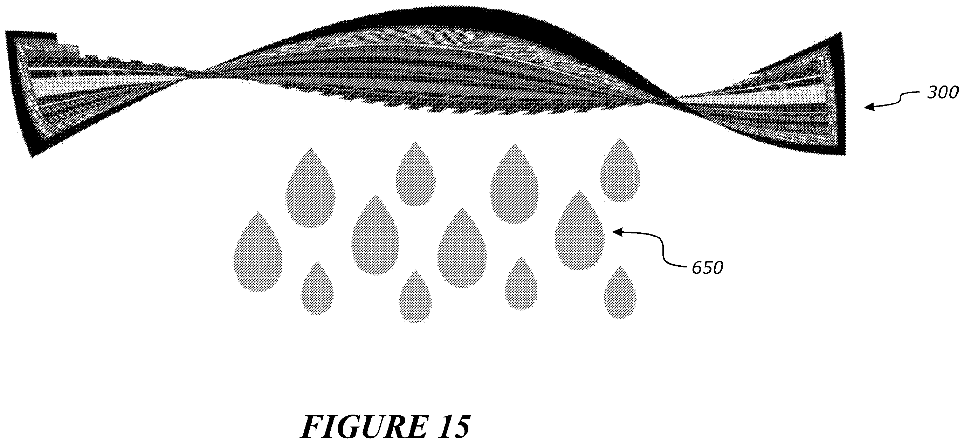

Once all the liquid is transferred to the second layer 320, the textile assembly 300 may act as a one-way system and may not let liquid through back up even under direct body weight pressure. The textile assembly 300 will release the liquid when twisted (e.g. during washing cycles) as the density of the knitted fibres are distorted and pores are made wider for liquid to escape through. This is shown in Figure 15. When the textile assembly 300 is twisted, substantially vertical fibres of the middle section 330C will be stretched and such stretching of the substantially vertical fibres will pull at least some portion of the second section 330B thereby causing at least some of the pores of the second section 330B to be wider (and distorting the density the knitted fibres of the second section 330B) for the liquid to escape easily. In other words, when the textile assembly 300 is twisted, wrung or squeezed, the substantially vertically oriented fibres (or tension in the substantially vertically oriented fibres) causes at least a portion of the second section 330B to be pulled/stretched thereby causing at least some of the pores of the second section 330B to be wider and/or knitting density of the second section 130Bto be distorted for at least some of the liquid to escape from the textile assembly 300.. Figure 14 shows a direct vertical pressure being applied (in the direction as shown by the arrow 800) to the textile assembly 300. The liquid 600 is retained within the second layer 320.

Once all the liquid is transferred to the second layer 320, the textile assembly 300 may act as a one-way system and may not let liquid through back up even under direct body weight pressure.

The textile assembly 300 will release the liquid when twisted (e.g. during washing cycles) as the density of the knitted fibres are distorted and pores are made wider for liquid to escape through. This is shown in Figure 15.

It will be appreciated that the textile assemblies 100, 200 as described above may also work in a similar manner as described above.

The leakproof layer 340 is preferably woven and treated with resin to retain all (or at least most) liquid within the second layer 320. In this example, the product is an underwear crotch with leakproof layer being located on the bottom and sides of the underwear crotch 600 therefore, the leakproof layer 340 in this example will retain all (or at least most) of the liquid from the bottom and sides of the underwear crotch 600. Consequently, the outer layer 350 is also protected.

Similar to the textile assemblies 100 and 200, the textile assembly 300 may be used in clothing such as but not limited to garments which may also include undergarments, sportswear etc. For example, the textile assembly 300 may be able to or is incorporated with or adapted for use as part of or is a garment. The textile assembly 300 may also be used in or as cleaning cloths, shoe inserts, shoes, sleep mats, place mats, inserts for brasseries, towels, mattress covers, feminine hygiene products, diapers, bags, sweat bands, nursing pads, underwear (e.g. male, female and children underwear), seat covers, bed sheets, maternity products and other products to absorb and retain any discharged liquids.

It will of course be realised that while the foregoing has been given by way of illustrative example of the present invention, all such modifications and variations thereto as would be apparent to a person skilled in the art are deemed to fall within the broad scope and ambit of the various aspects of invention as is hereinbefore described and/or defined in the claims.

### Advantages

One or more of the advantages of the present invention may be as follows:
- Simpler design
- Hypoallergenic
- Faster absorbency speed and drying properties
- Antibacterial
- Excellent liquid retention under pressure
- Higher absorbency
- Odour-proof
- One way leakproof but breathable
- Can last more than 200wash cycles without compromise on performance
- Can be used in or to make articles of clothing, e.g. undergarments
- Plastic free- Environment friendly
- Free of any harmful or hazardous chemicals
- Can be washed and tumbled dried at warm temperatures.
- Absorbency speed, levels & liquid retention can be achieved by. Warp spacer layer. Since the warp spacer is a combination of horizontal and vertical knit structures creating a 3D spacer layer that can transfer liquid from the first layer (quick dry layer) into the second layer (absorbent and retaining textile). The warp knitted structure has good liquid conductor properties compared to some other fabric structures. Because of the air space it defines, it also allows garments such as underwear to breath better, increase drying speed and reduce heat during wear.
- Warp knitted structure of the present invention also acts as barrier for the liquid to come back up under direct pressure because of its vertical/substantially vertical orientation but it will release liquid when the vertical/substantially vertical structure becomes distorted by twist motion.

## Claims

1. A multi-layer liquid absorbing and retaining textile assembly (100, 200, 300), the textile assembly comprising:
a first layer (110, 210, 310) made of or constructed as a pique knitted, one-way wicking material,
a second layer (120, 220, 320) that is tricot knitted and is made of or constructed as a microfibre material, and
an intermediate layer (130, 230, 330) made of or constructed as a warp knitted spacer fabric having a plurality of sections (130A, 130B, 130C; 230A, 230B, 230C; 330A, 330B, 330C) with at least one of the plurality of sections (130A, 230A, 330A) being proximal to the first layer and at least another one of the plurality of sections (130B, 230B, 330B) being proximal to the second layer,
the intermediate layer (130, 230, 330) being positioned between the first layer (110, 210, 310) and the second layer (120, 220, 320) and is adapted to interconnect the first layer and the second layer,
the first layer being adapted to transfer liquid from a first side (100A) of the textile assembly (100, 200, 300) to the intermediate layer (130, 230, 330), the intermediate layer is adapted to receive the liquid from the first layer (110, 210, 310) and the second layer (120, 220, 320) is adapted to retain the liquid at or proximal to a second side (100B) of the textile assembly (100, 200, 300) that is oppositely facing to the first side (100A),
wherein the plurality of sections comprise at least three sections that are a first section, a second section and a middle section with the middle section (130C, 230C, 330C) interconnecting the first (130A, 230A, 330A) and second (130B, 230B, 330B) sections, the first section (130A, 230A, 330A) being proximal to the first layer (110, 210, 310) and the second section (130B, 230B, 330B) being proximal to the second layer (120, 220, 320), wherein the knitting density of the first, second and middle sections are high to low in the following order:
ii. the second section (130B, 230B, 330B),
ii. the first section (130A, 230A, 330A),
iii. the middle section (130C, 230C, 330C),
wherein the first section (130A, 230A, 330A) is warp knitted to form a plurality of pores to receive the liquid from the first layer, wherein the middle section (130C, 230C, 330C) is warp knitted to form a substantially vertically oriented fibres, and wherein the second section (130B, 230B, 330B) is warp knitted to form a plurality of pores to receive the liquid from the middle section, wherein pores of the second section are smaller in size than the pores of the first section.

2. A textile assembly (100, 200, 300) as claimed in claim 1, wherein each of the plurality of sections has a knitting density that is different from a knitting density of another section or sections.

3. A textile assembly (100, 200, 300) as claimed in claim 1 or 2, wherein the liquid transferred from the first layer (110, 210, 310) is adapted to be received by the first section (130A), the middle section (130C, 230C, 330C) and the second section (130B, 230B, 330B) of the intermediate layer (130, 230, 330) in the following sequential order before being transferred to the second layer:
i. the first section (130A, 230A, 330A),
ii. the middle section (130C, 230C, 330C),
iii. the second section (130B, 230B, 330B).

4. A textile assembly (100, 200, 300) as claimed in any one of the preceding claims, wherein the microfibre material comprises a plurality of gaps in between for collecting liquid.

5. A textile assembly as claimed in any one of preceding claims, wherein the textile assembly (300) comprises a leakproof layer (340) sealing and/or trapping liquid at the second layer (320) at least on a side of the second layer that is opposite to side of the second layer (320) that is proximal to the intermediate layer (330).

6. A method of making a multi-layer liquid absorbing and retaining textile assembly for absorbing and retaining liquid emitted from a human body as defined in any one of the claims 1-5, the method comprising:
providing a first layer made of or constructed as a pique-knitted, one-way wicking material,
providing a second layer that is tricot knitted and is made of or constructed as a microfibre material,
providing an intermediate layer that is made of or constructed as warp knitted spacer fabric having a plurality of sections with at least one of the plurality of sections being configured to be proximal to the first layer and at least another one of the plurality of sections being proximal to the second layer,
positioning the intermediate layer between the first layer and the second layer and directly or indirectly affixing the intermediate layer to the first layer and the second layer so that the intermediate layer interconnects the first layer and the second layer.

## Patentansprüche

1. Mehrschichtige flüssigkeitsabsorbierende und -haltende Textilanordnung (100, 200, 300), wobei die Textilanordnung umfasst:
eine erste Schicht (110, 210, 310), die aus Piqué-gestricktem unidirektionalem Dochtmaterial gefertigt oder als solches konstruiert ist,
eine zweite Schicht (120, 220, 320), die Trikot-gestrickt ist und aus Mikrofasermaterial gefertigt oder als solches konstruiert ist, und
eine Zwischenschicht (130, 230, 330), die aus kettengewirktem Abstandsgewirk mit einer Vielzahl von Abschnitten (130A, 130B, 130C; 230A, 230B, 230C; 330A, 330B, 330C) gefertigt oder als solches konstruiert ist, wobei mindestens einer von der Vielzahl der Abschnitte (130A, 230A, 330A) sich proximal der ersten Schicht befindet und mindestens ein anderer von der Vielzahl der Abschnitte (130B, 230B, 330B) sich proximal der zweiten Schicht befindet,
die Zwischenschicht (130, 230, 330) zwischen der ersten Schicht (110, 210, 310) und der zweiten Schicht (120, 220, 320) positioniert ist und vorgesehen ist, um die erste Schicht und die zweite Schicht zu verbinden,
die erste Schicht vorgesehen ist, um Flüssigkeit von einer ersten Seite (100A) der Textilanordnung (100, 200, 300) zu der Zwischenschicht (130, 230, 330) zu transferieren, wobei die Zwischenschicht vorgesehen ist, um die Flüssigkeit von der ersten Schicht (110, 210, 310) zu empfangen, und die zweite Schicht (120, 220, 320) vorgesehen ist, um die Flüssigkeit an oder proximal einer zweiten Seite (100B) der Textilanordnung (100, 200, 300) zu halten, die zu der Gegenseite der ersten Seite (100A) weist,
wobei die Vielzahl der Abschnitte mindestens drei Abschnitte umfasst, die ein erster Abschnitt, ein zweiter Abschnitt und ein Mittelabschnitt sind, wobei der Mittelabschnitt (130C, 230C, 330C) den ersten (130A, 230A, 330A) und zweiten (130B, 230B, 330B) Abschnitt verbindet, der erste Abschnitt (130A, 230A, 330A) sich proximal der ersten Schicht (110, 210, 310) befindet, und der zweite Abschnitt (130B, 230B, 330B) sich proximal der zweiten Schicht (120, 220, 320) befindet, wobei die Strickdichte des ersten, zweiten und Mittelabschnitts in der folgenden Reihenfolge von hoch zu niedrig ist:
i. der zweite Abschnitt (130B, 230B, 330B),
ii. der erste Abschnitt (130A, 230A, 330A),
iii. der Mittelabschnitt (130C, 230C, 330C),
wobei der erste Abschnitt (130A, 230A, 330A) kettengewirkt ist, um eine Vielzahl von Poren zu bilden, um die Flüssigkeit von der ersten Schicht zu empfangen, wobei der Mittelabschnitt (130C, 230C, 330C) kettengewirkt ist, um im Wesentlichen vertikal orientierte Fasern zu bilden, und wobei der zweite Abschnitt (130B, 230B, 330B) kettengewirkt ist, um eine Vielzahl von Poren zu bilden, um die Flüssigkeit von dem Mittelabschnitt zu empfangen, wobei Poren des zweiten Abschnitts eine geringere Größe als die Poren des ersten Abschnitts haben.

2. Textilanordnung (100, 200, 300) nach Anspruch 1, wobei jeder von der Vielzahl der Abschnitte eine Strickdichte hat, die sich von der Strickdichte eines anderen Abschnitts oder von anderen Abschnitten unterscheidet.

3. Textilanordnung (100, 200, 300) nach Anspruch 1 oder 2, wobei vorgesehen ist, dass die von der ersten Schicht (110, 210, 310) transferierte Flüssigkeit von dem ersten Abschnitt (130A), dem Mittelabschnitt (130C, 230C, 330C) und dem zweiten Abschnitt (130B, 230B, 330B) der Zwischenschicht (130, 230, 330) in der folgenden sequentiellen Reihenfolge empfangen wird, bevor sie an die zweite Schicht transferiert wird:
i. dem ersten Abschnitt (130A, 230A, 330A),
ii. dem Mittelabschnitt (130C, 230C, 330C),
iii. dem zweiten Abschnitt (130B, 230B, 330B).

4. Textilanordnung (100, 200, 300) nach einem der vorhergehenden Ansprüche, wobei das Mikrofasermaterial eine Vielzahl von Lücken dazwischen umfasst, um Flüssigkeit zu sammeln.

5. Textilanordnung nach einem der vorhergehenden Ansprüche, wobei die Textilanordnung (300) eine lecksichere Schicht (340) umfasst, die Flüssigkeit an der zweiten Schicht (320) mindestens auf einer Seite der zweiten Schicht versiegelt und/oder abfängt, die der Seite der zweiten Schicht (320) gegenüber liegt, die proximal der Zwischenschicht (330) ist.

6. Verfahren zur Herstellung einer mehrschichtigen flüssigkeitsabsorbierenden und -haltenden Textilanordnung zum Absorbieren und Halten von Flüssigkeit, die von einem menschlichen Körper abgesondert wurde, welche in einem der Ansprüche 1 bis 5 definiert ist, wobei das Verfahren umfasst:
Bereitstellen einer ersten Schicht, die aus Piqué-gestricktem unidirektionalem Dochtmaterial gefertigt oder als solches konstruiert ist,
Bereitstellen einer zweiten Schicht, die Trikot-gestrickt ist und aus Mikrofasermaterial gefertigt oder als solches konstruiert ist, und
Bereitstellen einer Zwischenschicht, die aus kettengewirktem Abstandsgewirk mit einer Vielzahl von Abschnitten gefertigt oder als solches konstruiert ist, wobei mindestens einer von der Vielzahl der Abschnitte ausgestaltet ist, um sich proximal der ersten Schicht zu befinden, und mindestens ein anderer von der Vielzahl der Abschnitte sich proximal der zweiten Schicht befindet,
Positionieren der Zwischenschicht zwischen der ersten Schicht und der zweiten Schicht, und direktes oder indirektes Fixieren der Zwischenschicht an der ersten Schicht und der zweiten Schicht, so dass die Zwischenschicht die erste Schicht und die zweite Schicht verbindet.

## Revendications

1. Ensemble textile multicouche absorbant et retenant les liquides (100, 200, 300), l'ensemble textile comprenant :
une première couche (110, 210, 310) faite de ou construite sous la forme d'un matériau tricoté en piqué à effet de mèche unidirectionnel,
une deuxième couche (120, 220, 320) qui est tricotée et faite de ou construite sous la forme d'un matériau en microfibres, et
une couche intermédiaire (130, 230, 330) faite de ou construite sous la forme d'un tissu d'espacement tricoté en îne présentant une pluralité de sections (130A, 130B, 130C ; 230A, 230B, 230C ; 330A, 330B, 330C) avec au moins une de la pluralité de sections (130A, 230A, 330A) étant proximale à la première couche et au moins une autre de la pluralité de sections (130B, 230B, 330B) étant proximale à la deuxième couche,
la couche intermédiaire (130, 230, 330) étant positionnée entre la première couche (110, 210, 310) et la deuxième couche (120, 220, 320) et est adaptée pour interconnecter la première couche et la deuxième couche,
la première couche étant adaptée pour transférer le liquide d'un premier côté (100A) de l'ensemble textile (100, 200, 300) vers la couche intermédiaire (130, 230, 330), la couche intermédiaire est adaptée pour recevoir le liquide depuis la première couche (110, 210, 310) et la deuxième couche (120, 220, 320) est adaptée pour retenir le liquide au niveau ou à proximité d'un deuxième côté (100B) de l'ensemble textile (100, 200, 300) opposé au premier côté (100A),
dans lequel la pluralité de sections comprend au moins trois sections qui sont une première section, une deuxième section et une section médiane avec la section médiane (130C, 230C, 330C) interconnectant les première (130A, 230A, 330A) et deuxième (130B, 230B, 330B) sections, la première section (130A, 230A, 330A) étant proximale à la première couche (110, 210, 310) et la deuxième section (130B, 230B, 330B) étant proximale à la deuxième couche (120, 220, 320), dans lequel les densités de tricotage des sections première, deuxième et médiane sont élevées à faibles dans l'ordre suivant :
ii. la deuxième section (130B, 230B, 330B),
ii. la première section (130A, 230A, 330A),
iii. la section médiane (130C, 230C, 330C),
dans lequel la première section (130A, 230A, 330A) est tricotée en chaîne pour former une pluralité de pores pour recevoir le liquide depuis la première couche, dans lequel la section médiane (130C, 230C, 330C) est tricotée en chaîne pour former des fibres orientées sensiblement verticalement, et dans lequel la deuxième section (130B, 230B, 330B) est tricotée en chaîne pour former une pluralité de pores pour recevoir le liquide depuis la section médiane, dans lequel les pores de la deuxième section présentent une taille plus petite que les pores de la première section.

2. Ensemble textile (100, 200, 300) selon la revendication 1, dans lequel chacune de la pluralité de sections présente une densité de tricotage différente d'une densité de tricotage d'une ou d'autres section(s).

3. Ensemble textile (100, 200, 300) selon la revendication 1 ou 2, dans lequel le liquide transféré depuis la première couche (110, 210, 310) est adapté pour être reçu par la première section (130A), la section médiane (130C, 230C, 330C) et la deuxième section (130B, 230B, 330B) de la couche intermédiaire (130, 230, 330) dans l'ordre séquentiel suivant avant d'être transféré vers la deuxième couche :
i. la première section (130A, 230A, 330A),
ii. la section médiane (130C, 230C, 330C),
iii. la deuxième section (130B, 230B, 330B).

4. Ensemble textile (100, 200, 300) selon l'une quelconque des revendications précédentes, dans lequel le matériau en microfibres comprend une pluralité d'espaces entre eux pour collecter le liquide.

5. Ensemble textile selon l'une quelconque des revendications précédentes, dans lequel l'ensemble textile (300) comprend une couche étanche (340) scellant et/ou emprisonnant le liquide au niveau de la deuxième couche (320) au moins sur un côté de la deuxième couche opposé au côté de la deuxième couche (320) proximal à la couche intermédiaire (330).

6. Procédé de réalisation d'un ensemble textile multicouche absorbant et retenant un liquide pour absorber et retenir le liquide émis par un corps humain comme défini dans l'une quelconque des revendications 1-5, le procédé comprenant:
fournir une première couche faite de ou construite sous la forme d'un matériau tricoté en piqué à effet de mèche unidirectionnel,
fournir une deuxième couche qui est tricotée et faite de ou construite sous la forme d'un matériau en microfibres,
fournir une couche intermédiaire faite de ou construite sous la forme d'un tissu d'espacement tricoté en chaîne présentant une pluralité de sections avec au moins une de la pluralité de sections étant configurée pour être proximale à la première couche et au moins une autre de la pluralité de sections étant proximale à la deuxième couche,
positionner la couche intermédiaire entre la première couche et la deuxième couche et fixer directement ou indirectement la couche intermédiaire à la première couche et à la deuxième couche de telle sorte que la couche intermédiaire interconnecte la première couche et la deuxième couche.
